# EUROPEAN PATENT SPECIFICATION

(11) **EP 2 521 563 B1**
(45) Date of publication and mention of the grant of the patent: **07.12.2016**
(21) Application number: 11732058.0
(22) Date of filing: 05.01.2011
(51) Int. Cl.: A61K 38/16, A61K 39/02, A61K 9/127, A61P 37/00, A61P 31/12, C07K 14/11, C07K 14/00

(54) **COMPOSITIONS FOR PROVIDING PROTECTIVE IMMUNITY IN THE ELDERLY**
ZUSAMMENSETZUNGEN ZUR BEREITSTELLUNG VON SCHUTZIMMUNITÄT IM ALTER
COMPOSITIONS VISANT À CONFÉRER UNE IMMUNITÉ PROTECTRICE AUX PERSONNES ÂGÉES

(30) Priority: 06.01.2010 US 292593 P
(43) Date of publication of application: 14.11.2012
(73) Proprietor: VaxInnate Corporation, Cranbury, NJ 08512 (US)
(72) Inventor: TAYLOR, N., David, Cranbury NJ 08512 (US); BECKER, Robert, S., Cranbury NJ 08512 (US); SHAW, Alan, R., Cranbury NJ 08512 (US); TUSSEY, Lynda, G., Cranbury NJ 08512 (US)
(74) Representative: Kirkham, Nicholas Andrew
(86) International application number: PCT/US2011/020159
(87) International publication number: WO 2011/084967

(56) References cited:
- WO-A2-2009/128949
- US-A- 5 976 552
- US-A1- 2007 122 421
- US-A1- 2008 124 361
- US-A1- 2009 028 889
- US-A1- 2009 297 552
- HULEATT ET AL: "Potent immunogenicity and efficacy of a universal influenza vaccine candidate comprising a recombinant fusion protein linking influenza M2e to the TLR5 ligand flagellin", VACCINE, ELSEVIER LTD, GB, vol. 26, no. 2, 20 November 2007 (2007-11-20), pages 201-214, XP022394779, ISSN: 0264-410X, DOI: 10.1016/J.VACCINE.2007.10.062
- WANG BAO-ZHONG ET AL: "Incorporation of membrane-anchored flagellin into influenza virus-like particles enhances the breadth of immune responses.", JOURNAL OF VIROLOGY DEC 2008, vol. 82, no. 23, December 2008 (2008-12), pages 11813-11823, XP009170384, ISSN: 1098-5514
- PODDA ET AL: "The adjuvanted influenza vaccines with novel adjuvants: experience with the MF59-adjuvanted vaccine", VACCINE, ELSEVIER LTD, GB, vol. 19, no. 17-19, 21 March 2001 (2001-03-21), pages 2673-2680, XP027349991, ISSN: 0264-410X [retrieved on 2001-03-21]
- CUADROS C ET AL: "Flagellin fusion proteins as adjuvants or vaccines induce specific immune responses", INFECTION AND IMMUNITY, AMERICAN SOCIETY FOR MACROBIOLOGY, USA, vol. 72, no. 5, 1 May 2004 (2004-05-01), pages 2810-2816, XP002392967, ISSN: 0019-9567, DOI: 10.1128/IAI.72.5.2810-2816.2004

### SEQUENCE LISTING

<110> VaxInnate Corporation
<120> Methods and Compositions for Providing Protective Immunity in the Elderly
<130> VAX-ELDFLU
<150> 61/292,593
   <151> 2010-01-06
<160> 7
<170> PatentIn version 3.5
<210> 1
   <211> 727
   <212> PRT
   <213> Escherichia coli
<400> 1
   Ala Gln Val Ile Asn Thr Asn Ser Leu Ser Leu Leu Thr Gln Asn Asn
   1               5                   10                  15
   Leu Asn Lys Ser Gln Ser Ala Leu Gly Thr Ala Ile Glu Arg Leu Ser
               20                  25                  30
   Ser Gly Leu Arg Ile Asn Ser Ala Lys Asp Asp Ala Ala Gly Gln Ala
           35                  40                  45
   Ile Ala Asn Arg Phe Thr Ala Asn Ile Lys Gly Leu Thr Gln Ala Ser
       50                  55                  60
   Arg Asn Ala Asn Asp Gly Ile Ser Ile Ala Gln Thr Thr Glu Gly Ala
   65                  70                  75                  80
   Leu Asn Glu Ile Asn Asn Asn Leu Gln Arg Val Arg Glu Leu Ala Val
                   85                  90                  95
   Gln Ser Ala Asn Ser Thr Asn Ser Gln Ser Asp Leu Asp Ser Ile Gln
               100                 105                 110
   Ala Glu Ile Thr Gln Arg Leu Asn Glu Ile Asp Arg Val Ser Gly Gln
           115                 120                 125
   Thr Gln Phe Asn Gly Val Lys Val Leu Ala Gln Asp Asn Thr Leu Thr
       130                 135                 140
   Ile Gln Val Gly Ala Asn Asp Gly Glu Thr Ile Asp Ile Asp Leu Lys
   145                 150                 155                 160
   Gln Ile Asn Ser Gln Thr Leu Gly Leu Asp Ser Leu Asn Val Gln Lys
                   165                 170                 175
   Ala Tyr Asp Val Lys Asp Thr Ala Val Thr Thr Lys Ala Tyr Ala Asn
   180 185 190
   Asn Gly Thr Thr Leu Asp Val Ser Gly Leu Asp Asp Ala Ala Ile Lys
   195 200 205
   Ala Ala Thr Gly Gly Thr Asn Gly Thr Ala Ser Val Thr Gly Gly Ala
   210 215 220
   Val Lys Phe Asp Ala Asp Asn Asn Lys Tyr Phe Val Thr Ile Gly Gly
   225 230 235 240
   Phe Thr Gly Ala Asp Ala Ala Lys Asn Gly Asp Tyr Glu Val Asn Val
   245 250 255
   Ala Thr Asp Gly Thr Val Thr Leu Ala Ala Gly Ala Thr Lys Thr Thr
   260 265 270
   Met Pro Ala Gly Ala Thr Thr Lys Thr Glu Val Gln Glu Leu Lys Asp
   275 280 285
   Thr Pro Ala Val Val Ser Ala Asp Ala Lys Asn Ala Leu Ile Ala Gly
   290 295 300
   Gly Val Asp Ala Thr Asp Ala Asn Gly Ala Glu Leu Val Lys Met Ser
   305 310 315 320
   Tyr Thr Asp Lys Asn Gly Lys Thr Ile Glu Gly Gly Tyr Ala Leu Lys
   325 330 335
   Ala Gly Asp Lys Tyr Tyr Ala Ala Asp Tyr Asp Glu Ala Thr Gly Ala
   340 345 350
   Ile Lys Ala Lys Thr Thr Ser Tyr Thr Ala Ala Asp Gly Thr Thr Lys
   355 360 365
   Thr Ala Ala Asn Gln Leu Gly Gly Val Asp Gly Lys Thr Glu Val Val
   370 375 380
   Thr Ile Asp Gly Lys Thr Tyr Asn Ala Ser Lys Ala Ala Gly His Asp
   385 390 395 400
   Phe Lys Ala Gln Pro Glu Leu Ala Glu Ala Ala Ala Lys Thr Thr Glu
   405 410 415
   Asn Pro Leu Gln Lys Ile Asp Ala Ala Leu Ala Gln Val Asp Ala Leu
   420 425 430
   Arg Ser Asp Leu Gly Ala Val Gln Asn Arg Phe Asn Ser Ala Ile Thr
   435 440 445
   Asn Leu Gly Asn Thr Val Asn Asn Leu Ser Glu Ala Arg Ser Arg Ile
   450 455 460
   Glu Asp Ser Asp Tyr Ala Thr Glu Val Ser Asn Met Ser Arg Ala Gln
   465 470 475 480
   Ile Leu Gln Gln Ala Gly Thr Ser Val Leu Ala Gln Ala Asn Gln Val
   485 490 495
   Pro Gln Asn Val Leu Ser Leu Leu Ala Lys Gly Ile Ala Pro Leu Gln
   500 505 510
   Leu Gly Asn Cys Ser Val Ala Gly Trp Ile Leu Gly Asn Pro Glu Cys
   515 520 525
   Glu Leu Leu Ile Ser Arg Glu Ser Trp Ser Tyr Ile Val Glu Lys Pro
   530 535 540
   Asn Pro Glu Asn Gly Thr Cys Tyr Pro Gly His Phe Ala Asp Tyr Glu
   545 550 555 560
   Glu Leu Arg Glu Gln Leu Ser Ser Val Ser Ser Phe Glu Arg Phe Glu
   565 570 575
   Ile Phe Pro Lys Glu Ser Ser Trp Pro Asn His Thr Thr Thr Gly Val
   580 585 590
   Ser Ala Ser Cys Ser His Asn Gly Glu Ser Ser Phe Tyr Lys Asn Leu
   595 600 605
   Leu Trp Leu Thr Gly Lys Asn Gly Leu Tyr Pro Asn Leu Ser Lys Ser
   610 615 620
   Ala Asn Asn Lys Glu Lys Glu Val Leu Val Leu Trp Gly Val His His
   625 630 635 640
   Pro Pro Asn Ile Gly Asp Gln Arg Ala Leu Tyr His Lys Glu Asn Ala
   645 650 655
   Tyr Val Ser Val Val Ser Ser His Tyr Ser Arg Lys Phe Thr Pro Glu
   660 665 670
   Ile Ala Lys Arg Pro Lys Val Arg Asp Gln Glu Gly Arg Ile Asn Tyr
   675 680 685
   Tyr Trp Thr Leu Leu Glu Pro Gly Asp Thr Ile Ile Phe Glu Ala Asn
   690 695 700
   Gly Asn Leu Ile Ala Pro Arg Tyr Ala Phe Ala Leu Ser Arg Gly Phe
   705 710 715 720
   Gly Ser Gly Ile Ile Asn Ser
   725
<210> 2
   <211> 2193
   <212> DNA
   <213> Escherichia coli
<400> 2
   atggcacaag taatcaacac taacagtctg tcgctgctga cccagaataa cctgaacaaa      60
   tcccagtccg cactgggcac cgctatcgag cgtctgtctt ctggtctgcg tatcaacagc     120
   gcgaaagacg atgcggcagg tcaggcgatt gctaaccgtt tcaccgcgaa catcaaaggt     180
   ctgactcagg cttcccgtaa cgctaacgac ggtatctcca ttgcgcagac cactgaaggc     240
   gcgctgaacg aaatcaacaa caacctgcag cgtgtgcgtg aactggcggt tcagtctgct     300
   aacagcacca actcccagtc tgacctcgac tccatccagg ctgaaatcac ccagcgcctg     360
   aacgaaatcg accgtgtatc cggccagact cagttcaacg gcgtgaaagt cctggcgcag     420
   gacaacaccc tgaccatcca ggttggcgcc aacgacggtg aaactatcga tatcgatctg     480
   aagcagatca actctcagac cctgggtctg gactcactga acgtgcagaa agcgtatgat     540
   gtgaaagata cagcagtaac aacgaaagct tatgccaata atggtactac actggatgta     600
   tcgggtcttg atgatgcagc tattaaagcg gctacgggtg gtacgaatgg tacggcttct     660
   gtaaccggtg gtgcggttaa atttgacgca gataataaca agtactttgt tactattggt     720
   ggctttactg gtgctgatgc cgccaaaaat ggcgattatg aagttaacgt tgctactgac     780
   ggtacagtaa cccttgcggc tggcgcaact aaaaccacaa tgcctgctgg tgcgacaact     840
   aaaacagaag tacaggagtt aaaagataca ccggcagttg tttcagcaga tgctaaaaat     900
   gccttaattg ctggcggcgt tgacgctacc gatgctaatg gcgctgagtt ggtcaaaatg     960
   tcttataccg ataaaaatgg taagacaatt gaaggcggtt atgcgcttaa agctggcgat    1020
   aagtattacg ccgcagatta cgatgaagcg acaggagcaa ttaaagctaa aactacaagt    1080
   tatactgctg ctgacggcac taccaaaaca gcggctaacc aactgggtgg cgtagacggt    1140
   aaaaccgaag tcgttactat cgacggtaaa acctacaatg ccagcaaagc cgctggtcat    1200
   gatttcaaag cacaaccaga gctggcggaa gcagccgcta aaaccaccga aaacccgctg    1260
   cagaaaattg atgccgcgct ggcgcaggtg gatgcgctgc gctctgatct gggtgcggta    1320
   caaaaccgtt tcaactctgc tatcaccaac ctgggcaata ccgtaaacaa tctgtctgaa    1380
   gcgcgtagcc gtatcgaaga ttccgactac gcgaccgaag tttccaacat gtctcgcgcg    1440
   cagattctgc agcaggccgg tacttccgtt ctggcgcagg ctaaccaggt cccgcagaac    1500
   gtgctgtctc tgttagcgag cggcgtcgca ccgctgcatc tgggtaagtg taacattgct    1560
   ggttggattc tgggtaaccc agagtgtgag agcctgtcca ccgcgagcag ctggagctat    1620
   atcgtcgaaa ccccgagcag cgacaatggc acctgctatc ctggcgactt cattgattac    1680
   gaagaactgc gtgagcaact gagcagcgtg tcgagctttg agcgttttga aatcttcccg    1740
   aaaaccagca gctggccgaa tcatgacagc aataagggtg tcacggcagc gtgcccgcac    1800
   gctggtgcga aatcctttta caaaaacttg atttggctgg tgaagaaagg caatagctac    1860
   ccgaaactgt ctaagtccta catcaatgat aagggtaaag aagttctggt tctgtggggc    1920
   atccaccacc cgagcacttc tgcggatcaa caatctctgt accagaacgc cgacgcctac    1980
   gttttcgttg gcagctcccg ttacagcaag aaattcaaac cggagattgc gattcgcccg    2040
   aaggtccgtg accaggaagg tcgcatgaac tactattgga ccctggtgga gccgggtgat    2100
   aagatcacgt ttgaagccac gggtaacttg gtggttccgc gctatgcgtt cgcgatggag    2160
   cgtaacgcag gcagcggtat catcatttcg tag                                 2193
<210> 3
   <211> 730
   <212> PRT
   <213> Escherichia coli
<400> 3
   Met Ala Gln Val Ile Asn Thr Asn Ser Leu Ser Leu Leu Thr Gln Asn
   1               5                   10                  15
   Asn Leu Asn Lys Ser Gln Ser Ala Leu Gly Thr Ala Ile Glu Arg Leu
               20                  25                  30
   Ser Ser Gly Leu Arg Ile Asn Ser Ala Lys Asp Asp Ala Ala Gly Gln
           35                  40                  45
   Ala Ile Ala Asn Arg Phe Thr Ala Asn Ile Lys Gly Leu Thr Gln Ala
       50                  55                  60
   Ser Arg Asn Ala Asn Asp Gly Ile Ser Ile Ala Gln Thr Thr Glu Gly
   65                  70                  75                  80
   Ala Leu Asn Glu Ile Asn Asn Asn Leu Gln Arg Val Arg Glu Leu Ala
                   85                  90                  95
   Val Gln Ser Ala Asn Ser Thr Asn Ser Gln Ser Asp Leu Asp Ser Ile
               100                 105                 110
   Gln Ala Glu Ile Thr Gln Arg Leu Asn Glu Ile Asp Arg Val Ser Gly
           115                 120                 125
   Gln Thr Gln Phe Asn Gly Val Lys Val Leu Ala Gln Asp Asn Thr Leu
       130                 135                 140
   Thr Ile Gln Val Gly Ala Asn Asp Gly Glu Thr Ile Asp Ile Asp Leu
   145                 150                 155                 160
   Lys Gln Ile Asn Ser Gln Thr Leu Gly Leu Asp Ser Leu Asn Val Gln
                   165                 170                 175
   Lys Ala Tyr Asp Val Lys Asp Thr Ala Val Thr Thr Lys Ala Tyr Ala
               180                 185                 190
   Asn Asn Gly Thr Thr Leu Asp Val Ser Gly Leu Asp Asp Ala Ala Ile
           195                 200                 205
   Lys Ala Ala Thr Gly Gly Thr Asn Gly Thr Ala Ser Val Thr Gly Gly
       210                 215                 220
   Ala Val Lys Phe Asp Ala Asp Asn Asn Lys Tyr Phe Val Thr Ile Gly
   225                 230                 235                 240
   Gly Phe Thr Gly Ala Asp Ala Ala Lys Asn Gly Asp Tyr Glu Val Asn
                   245                 250                 255
   Val Ala Thr Asp Gly Thr Val Thr Leu Ala Ala Gly Ala Thr Lys Thr
               260                 265                 270
   Thr Met Pro Ala Gly Ala Thr Thr Lys Thr Glu Val Gln Glu Leu Lys
           275                 280                 285
   Asp Thr Pro Ala Val Val Ser Ala Asp Ala Lys Asn Ala Leu Ile Ala
       290                 295                 300
   Gly Gly Val Asp Ala Thr Asp Ala Asn Gly Ala Glu Leu Val Lys Met
   305                 310                 315                 320
   Ser Tyr Thr Asp Lys Asn Gly Lys Thr Ile Glu Gly Gly Tyr Ala Leu
                   325                 330                 335
   Lys Ala Gly Asp Lys Tyr Tyr Ala Ala Asp Tyr Asp Glu Ala Thr Gly
               340                 345                 350
   Ala Ile Lys Ala Lys Thr Thr Ser Tyr Thr Ala Ala Asp Gly Thr Thr
           355                 360                 365
   Lys Thr Ala Ala Asn Gln Leu Gly Gly Val Asp Gly Lys Thr Glu Val
       370                 375                 380
   Val Thr Ile Asp Gly Lys Thr Tyr Asn Ala Ser Lys Ala Ala Gly His
   385 390 395 400
   Asp Phe Lys Ala Gln Pro Glu Leu Ala Glu Ala Ala Ala Lys Thr Thr
   405 410 415
   Glu Asn Pro Leu Gln Lys Ile Asp Ala Ala Leu Ala Gln Val Asp Ala
   420 425 430
   Leu Arg Ser Asp Leu Gly Ala Val Gln Asn Arg Phe Asn Ser Ala Ile
   435 440 445
   Thr Asn Leu Gly Asn Thr Val Asn Asn Leu Ser Glu Ala Arg Ser Arg
   450 455 460
   Ile Glu Asp Ser Asp Tyr Ala Thr Glu Val Ser Asn Met Ser Arg Ala
   465 470 475 480
   Gln Ile Leu Gln Gln Ala Gly Thr Ser Val Leu Ala Gln Ala Asn Gln
   485 490 495
   Val Pro Gln Asn Val Leu Ser Leu Leu Ala Ser Gly Val Ala Pro Leu
   500 505 510
   His Leu Gly Lys Cys Asn Ile Ala Gly Trp Ile Leu Gly Asn Pro Glu
   515 520 525
   Cys Glu Ser Leu Ser Thr Ala Ser Ser Trp Ser Tyr Ile Val Glu Thr
   530 535 540
   Pro Ser Ser Asp Asn Gly Thr Cys Tyr Pro Gly Asp Phe Ile Asp Tyr
   545 550 555 560
   Glu Glu Leu Arg Glu Gln Leu Ser Ser Val Ser Ser Phe Glu Arg Phe
   565 570 575
   Glu Ile Phe Pro Lys Thr Ser Ser Trp Pro Asn His Asp Ser Asn Lys
   580 585 590
   Gly Val Thr Ala Ala Cys Pro His Ala Gly Ala Lys Ser Phe Tyr Lys
   595 600 605
   Asn Leu Ile Trp Leu Val Lys Lys Gly Asn Ser Tyr Pro Lys Leu Ser
   610 615 620
   Lys Ser Tyr Ile Asn Asp Lys Gly Lys Glu Val Leu Val Leu Trp Gly
   625 630 635 640
   Ile His His Pro Ser Thr Ser Ala Asp Gln Gln Ser Leu Tyr Gln Asn
   645 650 655
   Ala Asp Ala Tyr Val Phe Val Gly Ser Ser Arg Tyr Ser Lys Lys Phe
   660 665 670
   Lys Pro Glu Ile Ala Ile Arg Pro Lys Val Arg Asp Gln Glu Gly Arg
   675 680 685
   Met Asn Tyr Tyr Trp Thr Leu Val Glu Pro Gly Asp Lys Ile Thr Phe
   690 695 700
   Glu Ala Thr Gly Asn Leu Val Val Pro Arg Tyr Ala Phe Ala Met Glu
   705 710 715 720
   Arg Asn Ala Gly Ser Gly Ile Ile Ile Ser
   725 730
<210> 4
   <211> 1887
   <212> DNA
   <213> Escherichia coli
<400> 4
   atggcacaag taatcaacac taacagtctg tcgctgctga cccagaataa cctgaacaaa      60
   tcccagtccg cactgggcac cgctatcgag cgtctgtctt ctggtctgcg tatcaacagc     120
   gcgaaagacg atgcggcagg tcaggcgatt gctaaccgtt tcaccgcgaa catcaaaggt     180
   ctgactcagg cttcccgtaa cgctaacgac ggtatctcca ttgcgcagac cactgaaggc     240
   gcgctgaacg aaatcaacaa caacctgcag cgtgtgcgtg aactggcggt tcagtctgct     300
   aacagcacca actcccagtc tgacctcgac tccatccagg ctgaaatcac ccagcgcctg     360
   aacgaaatcg accgtgtatc cggccagact cagttcaacg gcgtgaaagt cctggcgcag     420
   gacaacaccc tgaccatcca ggttggcgcc aacgacggtg aaactatcga tatcgatctg     480
   aagcagatca actctcagac cctgggtctg gactcactga acgtgcagaa agcgtatgat     540
   gtgaaagata cagcagtaac aacgaaagct ggcgtcgcac cgctgcatct gggtaagtgt     600
   aacattgctg gttggattct gggtaaccca gagtgtgaga gcctgtccac cgcgagcagc     660
   tggagctata tcgtcgaaac cccgagcagc gacaatggca cctgctatcc tggcgacttc     720
   attgattacg aagaactgcg tgagcaactg agcagcgtgt cgagctttga gcgttttgaa     780
   atcttcccga aaaccagcag ctggccgaat catgacagca ataagggtgt cacggcagcg     840
   tgcccgcacg ctggtgcgaa atccttttac aaaaacttga tttggctggt gaagaaaggc     900
   aatagctacc cgaaactgtc taagtcctac atcaatgata agggtaaaga agttctggtt     960
   ctgtggggca tccaccaccc gagcacttct gcggatcaac aatctctgta ccagaacgcc    1020
   gacgcctacg ttttcgttgg cagctcccgt tacagcaaga aattcaaacc ggagattgcg    1080
   attcgcccga aggtccgtga ccaggaaggt cgcatgaact actattggac cctggtggag 1140
   ccgggtgata agatcacgtt tgaagccacg ggtaacttgg tggttccgcg ctatgcgttc 1200
   gcgatggagc gtaacgcagg cagcggtatc atcatttcgt cagttgtttc agcagatgct 1260
   aaaaatgcct taattgctgg cggcgttgac gctaccgatg ctaatggcgc tgagttggtc 1320
   aaaatgtctt ataccgataa aaatggtaag acaattgaag gcggttatgc gcttaaagct 1380
   ggcgataagt attacgccgc agattacgat gaagcgacag gagcaattaa agctaaaacc 1440
   acaagttata ctgctgctga cggcactacc aaaacagcgg ctaaccaact gggtggcgta 1500
   gacggtaaaa ccgaagtcgt tactatcgac ggtaaaacct acaatgccag caaagccgct 1560
   ggtcatgatt tcaaagcaca accagagctg gcggaagcag ccgctaaaac caccgaaaac 1620
   ccgctgcaga aaattgatgc cgcgctggcg caggtggatg cgctgcgctc tgatctgggt 1680
   gcggtacaaa accgtttcaa ctctgctatc accaacctgg gcaataccgt aaacaatctg 1740
   tctgaagcgc gtagccgtat cgaagattcc gactacgcga ccgaagtttc caacatgtct 1800
   cgcgcgcaga ttttgcagca ggccggtact tccgttctgg cgcaggctaa ccaggtcccg 1860
   cagaacgtgc tgagcctgtt acgttag 1887
<210> 5
   <211> 628
   <212> PRT
   <213> Escherichia coli
<400> 5
   Met Ala Gln Val Ile Asn Thr Asn Ser Leu Ser Leu Leu Thr Gln Asn
   1 5 10 15
   Asn Leu Asn Lys Ser Gln Ser Ala Leu Gly Thr Ala Ile Glu Arg Leu
   20 25 30
   Ser Ser Gly Leu Arg Ile Asn Ser Ala Lys Asp Asp Ala Ala Gly Gln
   35 40 45
   Ala Ile Ala Asn Arg Phe Thr Ala Asn Ile Lys Gly Leu Thr Gln Ala
   50 55 60
   Ser Arg Asn Ala Asn Asp Gly Ile Ser Ile Ala Gln Thr Thr Glu Gly
   65 70 75 80
   Ala Leu Asn Glu Ile Asn Asn Asn Leu Gln Arg Val Arg Glu Leu Ala
   85 90 95
   Val Gln Ser Ala Asn Ser Thr Asn Ser Gln Ser Asp Leu Asp Ser Ile
   100 105 110
   Gln Ala Glu Ile Thr Gln Arg Leu Asn Glu Ile Asp Arg Val Ser Gly
   115 120 125
   Gln Thr Gln Phe Asn Gly Val Lys Val Leu Ala Gln Asp Asn Thr Leu
   130 135 140
   Thr Ile Gln Val Gly Ala Asn Asp Gly Glu Thr Ile Asp Ile Asp Leu
   145 150 155 160
   Lys Gln Ile Asn Ser Gln Thr Leu Gly Leu Asp Ser Leu Asn Val Gln
   165 170 175
   Lys Ala Tyr Asp Val Lys Asp Thr Ala Val Thr Thr Lys Ala Gly Val
   180 185 190
   Ala Pro Leu His Leu Gly Lys Cys Asn Ile Ala Gly Trp Ile Leu Gly
   195 200 205
   Asn Pro Glu Cys Glu Ser Leu Ser Thr Ala Ser Ser Trp Ser Tyr Ile
   210 215 220
   Val Glu Thr Pro Ser Ser Asp Asn Gly Thr Cys Tyr Pro Gly Asp Phe
   225 230 235 240
   Ile Asp Tyr Glu Glu Leu Arg Glu Gln Leu Ser Ser Val Ser Ser Phe
   245 250 255
   Glu Arg Phe Glu Ile Phe Pro Lys Thr Ser Ser Trp Pro Asn His Asp
   260 265 270
   Ser Asn Lys Gly Val Thr Ala Ala Cys Pro His Ala Gly Ala Lys Ser
   275 280 285
   Phe Tyr Lys Asn Leu Ile Trp Leu Val Lys Lys Gly Asn Ser Tyr Pro
   290 295 300
   Lys Leu Ser Lys Ser Tyr Ile Asn Asp Lys Gly Lys Glu Val Leu Val
   305 310 315 320
   Leu Trp Gly Ile His His Pro Ser Thr Ser Ala Asp Gln Gln Ser Leu
   325 330 335
   Tyr Gln Asn Ala Asp Ala Tyr Val Phe Val Gly Ser Ser Arg Tyr Ser
   340 345 350
   Lys Lys Phe Lys Pro Glu Ile Ala Ile Arg Pro Lys Val Arg Asp Gln
   355 360 365
   Glu Gly Arg Met Asn Tyr Tyr Trp Thr Leu Val Glu Pro Gly Asp Lys
       370                 375                 380
   Ile Thr Phe Glu Ala Thr Gly Asn Leu Val Val Pro Arg Tyr Ala Phe
   385                 390                 395                 400
   Ala Met Glu Arg Asn Ala Gly Ser Gly Ile Ile Ile Ser Ser Val Val
                   405                 410                 415
   Ser Ala Asp Ala Lys Asn Ala Leu Ile Ala Gly Gly Val Asp Ala Thr
               420                 425                 430
   Asp Ala Asn Gly Ala Glu Leu Val Lys Met Ser Tyr Thr Asp Lys Asn
           435                 440                 445
   Gly Lys Thr Ile Glu Gly Gly Tyr Ala Leu Lys Ala Gly Asp Lys Tyr
       450                 455                 460
   Tyr Ala Ala Asp Tyr Asp Glu Ala Thr Gly Ala Ile Lys Ala Lys Thr
   465                 470                 475                 480
   Thr Ser Tyr Thr Ala Ala Asp Gly Thr Thr Lys Thr Ala Ala Asn Gln
                   485                 490                 495
   Leu Gly Gly Val Asp Gly Lys Thr Glu Val Val Thr Ile Asp Gly Lys
               500                 505                 510
   Thr Tyr Asn Ala Ser Lys Ala Ala Gly His Asp Phe Lys Ala Gln Pro
           515                 520                 525
   Glu Leu Ala Glu Ala Ala Ala Lys Thr Thr Glu Asn Pro Leu Gln Lys
       530                 535                 540
   Ile Asp Ala Ala Leu Ala Gln Val Asp Ala Leu Arg Ser Asp Leu Gly
   545                 550                 555                 560
   Ala Val Gln Asn Arg Phe Asn Ser Ala Ile Thr Asn Leu Gly Asn Thr
                   565                 570                 575
   Val Asn Asn Leu Ser Glu Ala Arg Ser Arg Ile Glu Asp Ser Asp Tyr
               580                 585                 590
   Ala Thr Glu Val Ser Asn Met Ser Arg Ala Gln Ile Leu Gln Gln Ala
           595                 600                 605
   Gly Thr Ser Val Leu Ala Gln Ala Asn Gln Val Pro Gln Asn Val Leu
       610                 615                 620
   Ser Leu Leu Arg
   625
<210> 6
   <211> 2559
   <212> DNA
   <213> Escherichia coli
<400> 6
   atggcacaag taatcaacac taacagtctg tcgctgctga cccagaataa cctgaacaaa      60
   tcccagtccg cactgggcac cgctatcgag cgtctgtctt ctggtctgcg tatcaacagc     120
   gcgaaagacg atgcggcagg tcaggcgatt gctaaccgtt tcaccgcgaa catcaaaggt     180
   ctgactcagg cttcccgtaa cgctaacgac ggtatctcca ttgcgcagac cactgaaggc     240
   gcgctgaacg aaatcaacaa caacctgcag cgtgtgcgtg aactggcggt tcagtctgct     300
   aacagcacca actcccagtc tgacctcgac tccatccagg ctgaaatcac ccagcgcctg     360
   aacgaaatcg accgtgtatc cggccagact cagttcaacg gcgtgaaagt cctggcgcag     420
   gacaacaccc tgaccatcca ggttggcgcc aacgacggtg aaactatcga tatcgatctg     480
   aagcagatca actctcagac cctgggtctg gactcactga acgtgcagaa agcgtatgat     540
   gtgaaagata cagcagtaac aacgaaagct ggcgtcgcac cgctgcatct gggtaagtgt     600
   aacattgctg gttggattct gggtaaccca gagtgtgaga gcctgtccac cgcgagcagc     660
   tggagctata tcgtcgaaac cccgagcagc gacaatggca cctgctatcc tggcgacttc     720
   attgattacg aagaactgcg tgagcaactg agcagcgtgt cgagctttga gcgttttgaa     780
   atcttcccga aaaccagcag ctggccgaat catgacagca ataagggtgt cacggcagcg     840
   tgcccgcacg ctggtgcgaa atccttttac aaaaacttga tttggctggt gaagaaaggc      900
   aatagctacc cgaaactgtc taagtcctac atcaatgata agggtaaaga agttctggtt     960
   ctgtggggca tccaccaccc gagcacttct gcggatcaac aatctctgta ccagaacgcc    1020
   gacgcctacg ttttcgttgg cagctcccgt tacagcaaga aattcaaacc ggagattgcg    1080
   attcgcccga aggtccgtga ccaggaaggt cgcatgaact actattggac cctggtggag    1140
   ccgggtgata agatcacgtt tgaagccacg ggtaacttgg tggttccgcg ctatgcgttc    1200
   gcgatggagc gtaacgcagg cagcggtatc atcatttcgt cagttgtttc agcagatgct    1260
   aaaaatgcct taattgctgg cggcgttgac gctaccgatg ctaatggcgc tgagttggtc    1320
   aaaatgtctt ataccgataa aaatggtaag acaattgaag gcggttatgc gcttaaagct    1380
   ggcgataagt attacgccgc agattacgat gaagcgacag gagcaattaa agctaaaact    1440
   acaagttata ctgctgctga cggcactacc aaaacagcgg ctaaccaact gggtggcgta    1500
   gacggtaaaa ccgaagtcgt tactatcgac ggtaaaacct acaatgccag caaagccgct    1560
   ggtcatgatt tcaaagcaca accagagctg gcggaagcag ccgctaaaac caccgaaaac    1620
   ccgctgcaga aaattgatgc cgcgctggcg caggtggatg cgctgcgctc tgatctgggt    1680
   gcggtacaaa accgtttcaa ctctgctatc accaacctgg gcaataccgt aaacaatctg    1740
   tctgaagcgc gtagccgtat cgaagattcc gactacgcga ccgaagtttc caacatgtct      1800
   cgcgcgcaga ttctgcagca ggccggtact tccgttctgg cgcaggctaa ccaggtcccg      1860
   cagaacgtgc tgtctctgtt agcgagcggc gtcgcaccgc tgcatctggg taagtgtaac      1920
   attgctggtt ggattctggg taacccagag tgtgagagcc tgtccaccgc gagcagctgg      1980
   agctatatcg tcgaaacccc gagcagcgac aatggcacct gctatcctgg cgacttcatt      2040
   gattacgaag aactgcgtga gcaactgagc agcgtgtcga gctttgagcg ttttgaaatc      2100
   ttcccgaaaa ccagcagctg gccgaatcat gacagcaata agggtgtcac ggcagcgtgc      2160
   ccgcacgctg gtgcgaaatc cttttacaaa aacttgattt ggctggtgaa gaaaggcaat      2220
   agctacccga aactgtctaa gtcctacatc aatgataagg gtaaagaagt tctggttctg      2280
   tggggcatcc accacccgag cacttctgcg gatcaacaat ctctgtacca gaacgccgac      2340
   gcctacgttt tcgttggcag ctcccgttac agcaagaaat tcaaaccgga gattgcgatt      2400
   cgcccgaagg tccgtgacca ggaaggtcgc atgaactact attggaccct ggtggagccg      2460
   ggtgataaga tcacgtttga agccacgggt aacttggtgg ttccgcgcta tgcgttcgcg      2520
   atggagcgta acgcaggcag cggtatcatc atttcgtag                              2559
<210> 7
   <211> 852
   <212> PRT
   <213> Escherichia coli
<400> 7
   Met Ala Gln Val Ile Asn Thr Asn Ser Leu Ser Leu Leu Thr Gln Asn
   1               5                   10                  15
   Asn Leu Asn Lys Ser Gln Ser Ala Leu Gly Thr Ala Ile Glu Arg Leu
               20                  25                  30
   Ser Ser Gly Leu Arg Ile Asn Ser Ala Lys Asp Asp Ala Ala Gly Gln
           35                  40                  45
   Ala Ile Ala Asn Arg Phe Thr Ala Asn Ile Lys Gly Leu Thr Gln Ala
       50                  55                  60
   Ser Arg Asn Ala Asn Asp Gly Ile Ser Ile Ala Gln Thr Thr Glu Gly
   65                  70                  75                  80
   Ala Leu Asn Glu Ile Asn Asn Asn Leu Gln Arg Val Arg Glu Leu Ala
                   85                  90                  95
   Val Gln Ser Ala Asn Ser Thr Asn Ser Gln Ser Asp Leu Asp Ser Ile
               100                 105                 110
   Gln Ala Glu Ile Thr Gln Arg Leu Asn Glu Ile Asp Arg Val Ser Gly
   115 120 125
   Gln Thr Gln Phe Asn Gly Val Lys Val Leu Ala Gln Asp Asn Thr Leu
   130 135 140
   Thr Ile Gln Val Gly Ala Asn Asp Gly Glu Thr Ile Asp Ile Asp Leu
   145 150 155 160
   Lys Gln Ile Asn Ser Gln Thr Leu Gly Leu Asp Ser Leu Asn Val Gln
   165 170 175
   Lys Ala Tyr Asp Val Lys Asp Thr Ala Val Thr Thr Lys Ala Gly Val
   180 185 190
   Ala Pro Leu His Leu Gly Lys Cys Asn Ile Ala Gly Trp Ile Leu Gly
   195 200 205
   Asn Pro Glu Cys Glu Ser Leu Ser Thr Ala Ser Ser Trp Ser Tyr Ile
   210 215 220
   Val Glu Thr Pro Ser Ser Asp Asn Gly Thr Cys Tyr Pro Gly Asp Phe
   225 230 235 240
   Ile Asp Tyr Glu Glu Leu Arg Glu Gln Leu Ser Ser Val Ser Ser Phe
   245 250 255
   Glu Arg Phe Glu Ile Phe Pro Lys Thr Ser Ser Trp Pro Asn His Asp
   260 265 270
   Ser Asn Lys Gly Val Thr Ala Ala Cys Pro His Ala Gly Ala Lys Ser
   275 280 285
   Phe Tyr Lys Asn Leu Ile Trp Leu Val Lys Lys Gly Asn Ser Tyr Pro
   290 295 300
   Lys Leu Ser Lys Ser Tyr Ile Asn Asp Lys Gly Lys Glu Val Leu Val
   305 310 315 320
   Leu Trp Gly Ile His His Pro Ser Thr Ser Ala Asp Gln Gln Ser Leu
   325 330 335
   Tyr Gln Asn Ala Asp Ala Tyr Val Phe Val Gly Ser Ser Arg Tyr Ser
   340 345 350
   Lys Lys Phe Lys Pro Glu Ile Ala Ile Arg Pro Lys Val Arg Asp Gln
   355 360 365
   Glu Gly Arg Met Asn Tyr Tyr Trp Thr Leu Val Glu Pro Gly Asp Lys
   370 375 380
   Ile Thr Phe Glu Ala Thr Gly Asn Leu Val Val Pro Arg Tyr Ala Phe
   385 390 395 400
   Ala Met Glu Arg Asn Ala Gly Ser Gly Ile Ile Ile Ser Ser Val Val
   405 410 415
   Ser Ala Asp Ala Lys Asn Ala Leu Ile Ala Gly Gly Val Asp Ala Thr
   420 425 430
   Asp Ala Asn Gly Ala Glu Leu Val Lys Met Ser Tyr Thr Asp Lys Asn
   435 440 445
   Gly Lys Thr Ile Glu Gly Gly Tyr Ala Leu Lys Ala Gly Asp Lys Tyr
   450 455 460
   Tyr Ala Ala Asp Tyr Asp Glu Ala Thr Gly Ala Ile Lys Ala Lys Thr
   465 470 475 480
   Thr Ser Tyr Thr Ala Ala Asp Gly Thr Thr Lys Thr Ala Ala Asn Gln
   485 490 495
   Leu Gly Gly Val Asp Gly Lys Thr Glu Val Val Thr Ile Asp Gly Lys
   500 505 510
   Thr Tyr Asn Ala Ser Lys Ala Ala Gly His Asp Phe Lys Ala Gln Pro
   515 520 525
   Glu Leu Ala Glu Ala Ala Ala Lys Thr Thr Glu Asn Pro Leu Gln Lys
   530 535 540
   Ile Asp Ala Ala Leu Ala Gln Val Asp Ala Leu Arg Ser Asp Leu Gly
   545 550 555 560
   Ala Val Gln Asn Arg Phe Asn Ser Ala Ile Thr Asn Leu Gly Asn Thr
   565 570 575
   Val Asn Asn Leu Ser Glu Ala Arg Ser Arg Ile Glu Asp Ser Asp Tyr
   580 585 590
   Ala Thr Glu Val Ser Asn Met Ser Arg Ala Gln Ile Leu Gln Gln Ala
   595 600 605
   Gly Thr Ser Val Leu Ala Gln Ala Asn Gln Val Pro Gln Asn Val Leu
   610 615 620
   Ser Leu Leu Ala Ser Gly Val Ala Pro Leu His Leu Gly Lys Cys Asn
   625 630 635 640
   Ile Ala Gly Trp Ile Leu Gly Asn Pro Glu Cys Glu Ser Leu Ser Thr
   645 650 655
   Ala Ser Ser Trp Ser Tyr Ile Val Glu Thr Pro Ser Ser Asp Asn Gly
   660 665 670
   Thr Cys Tyr Pro Gly Asp Phe Ile Asp Tyr Glu Glu Leu Arg Glu Gln
   675 680 685
   Leu Ser Ser Val Ser Ser Phe Glu Arg Phe Glu Ile Phe Pro Lys Thr
   690 695 700
   Ser Ser Trp Pro Asn His Asp Ser Asn Lys Gly Val Thr Ala Ala Cys
   705 710 715 720
   Pro His Ala Gly Ala Lys Ser Phe Tyr Lys Asn Leu Ile Trp Leu Val
   725 730 735
   Lys Lys Gly Asn Ser Tyr Pro Lys Leu Ser Lys Ser Tyr Ile Asn Asp
   740 745 750
   Lys Gly Lys Glu Val Leu Val Leu Trp Gly Ile His His Pro Ser Thr
   755 760 765
   Ser Ala Asp Gln Gln Ser Leu Tyr Gln Asn Ala Asp Ala Tyr Val Phe
   770 775 780
   Val Gly Ser Ser Arg Tyr Ser Lys Lys Phe Lys Pro Glu Ile Ala Ile
   785 790 795 800
   Arg Pro Lys Val Arg Asp Gln Glu Gly Arg Met Asn Tyr Tyr Trp Thr
   805 810 815
   Leu Val Glu Pro Gly Asp Lys Ile Thr Phe Glu Ala Thr Gly Asn Leu
   820 825 830
   Val Val Pro Arg Tyr Ala Phe Ala Met Glu Arg Asn Ala Gly Ser Gly
   835 840 845
   Ile Ile Ile Ser
   850

## Description

### Background of the invention

Many elderly humans (greater than 65 years of age) have been immunized or exposed to many diseases, such as influenza, yet many may still require immunization in order to remain healthy. The immune response to active immunization tends to decrease with age.

Improving the response to influenza vaccines has been a major initiative since the 1970s (Mostow SR, et al. (1973) Inactivate vaccines in Volunteer studies with very high doses of influenza vaccine purified by zonal centrifugation Postgrad Med. J. 49: 152-158). Numerous investigators have explored the effect of doses of hemagglutinin (HA) greater than the standard 15 µg dose. The most thorough and well documented effort to provide an efficacious flu vaccine for the elderly has involved FluZone^{®}. Three successive trials (Keitel et al. (2006) Safety of high doses of influenza vaccine and effect on antibody responses in elderly persons. Arch. Intern. Med. 166: 1121-1127 (Table 1); Couch et al. (2007) Safety and immunogenicity of a high dose trivalent influenza vaccine among elderly subjects Vaccine 25: 7656-7663 (Table 2) and Falsey et al., (2009) Randomized, double-blind controlled phase 3 trial comparing the immunogenicity of high-dose and standard-dose influenza vaccine in adults 65 years and older. J. Infect Dis. 200: 172-180 (Table 3)) have shown that increasing the dose of HA from 15 µg to 60 µg, focusing on the HA component, yields an improvement in HA1 titers of about 1.6 to 1.7 fold.

**Table 1. Keitel, Arch. Int. Med. 2006**

| | | HAI Titers | |
|---|---|---|---|
| H1 | Dose (µg) | Pre | Post |
| Std Vx | 15 | 20 | 37 |
| | 30 | 20 | 50 |
| | 60 | 22 | 61 |

**Table 2. Elderly, Couch, Vaccine 2007**

| | HAI Titers | | | HAI Titers | |
|---|---|---|---|---|---|
| H1 | All points pre | All points post | H1 | No prior vx pre | No prior vx post |
| Std 15 µg | 11 | 21 | Std 15 µg | 7.4 | 24 |
| Hi 60 µg | 9 | 36 | Hi 60 µg | 6.1 | 54 |

**Table 3. Falsey, Treanor, JID, 2009, Standard dose vs. 60µg in 3876 subjects**

| | | HAI Titers | | | | |
|---|---|---|---|---|---|---|
| H1 | Dose | Day 0 | Day 28 | % SC | %SP | Fold x |
| High | 60µg | 28.5 | 115.6 | 48.6 | 89.9 | 4.1 |
| Std | 15µg | 29.4 | 67.3 | 23.1 | 76.8 | 2.3 |
| VAX125 | 2µg | 24.6 | 69.6 | 35 | 70 | 2.8 |
| | 3µg | 51 | 149.3 | 40 | 95 | 2.9 |

More aggressive studies with doses as high as 405 µg (Keitel, et al. (1994) High doses of purified influenza A virus hemagglutinin significantly augment serum and nasal secretion antibody responses in healthy young adults. J Clin Microbiol 32: 2468-2473; (Table 4) Keitel et al. (1996) Increased doses of purified influenza hemagglutinin and subvirion vaccines enhance antibody responses in the elderly Clin Diagn Lab immunol 3: 507-510 (Table 5)) resulted in an approximately 2.1 fold improvement in HA1 titers.

**Table 4. Keitel, Couch, J. Clin. Microbiol. 1994, Bromelain Cleaved, Pure HA vs. Std. VX**

| | | HAI Titers | | | HAI Titers | | |
|---|---|---|---|---|---|---|---|
| H1 | Dose (µg) | Lo Pre | Lo Post | % SC | Hi Pre | Hi Post | %SC |
| Mono | 15 | 6 | 97 | 100 | 42 | 128 | 53 |
| Purified | 45 | 7 | 100 | 100 | 39 | 294 | 87 |
| | 135 | 6 | 100 | 100 | 37 | 274 | 87 |
| | 405 | 6 | 100 | 34 | 34 | 239 | 100 |
| Std VX | 15 | 20 | 55 | | | | |
| Tri-valent | 15 | 17 | 63 | | | | |
| Purified | 45 | 14 | 86 | | | | |
| | 135 | 16 | 73 | | | | |

**Table 5. Elderly, Keitel, Clin. Diag. Lab. 1996, Purified HA vs. Normal Subvirion**

| | | HAI Titers | |
|---|---|---|---|
| H1 | Dose (µg) | pre | Post |
| Subvirion | 15 | 34 | 42 |
| | 45 | 42 | 56 |
| | 135 | 32 | 84 |
| Purified | 15 | 36 | 42 |
| | 45 | 36 | 84 |
| | 135 | 22 | 120 |

Other studies demonstrating the effects of influenza vaccine in the elderly are shown in the tables below.

**Table 6 Increased dose or booster in Elderly, Cools et al. J. Med. Virol. 2009, 15µg, 15 + 15µg, 30µg, 30 + 15µg**

| | HAI Titers | | | | HAI Titers | | | |
|---|---|---|---|---|---|---|---|---|
| H3 only | Day 0 | Day 25 | SC% | SP% | Day 84 | Day 109 | SC% | SP% |
| 15 + pbo | 22.7 | 57.8 | 33.3 | 66.3 | | 49.3 | 21.7 | 63.9 |
| 15 + 15 | 22.2 | | | | 48.6 | 53.9 | 34.5 | 61.9 |
| 30 + pbo | 18.4 | 70.6 | 45.3 | 73.3 | | | 35.4 | 64.9 |
| 30+15 | 20.6 | | | | 53.5 | 66.4 | 43.6 | 74.2 |

**Table 7 AVP-MSD Vaxigrip vs. Fluad in Elderly, Squarcione, Sgricia, Biasio, Pernetti Vaccine 2003.**

| H1 | Vaxigrip | Fluad |
|---|---|---|
| Pre-GMT | 10.4 | 11.3 |
| Post d21 GMT | 87.1 | 154.4 |
| Seroprotection % | | |
| Under 75 years of age | 74.9 | 86.4 |
| Over 75 years of age | 66.1 | 78.9 |
| Whole population | 71.6 | 83.4 |
| Seroconversion | | |
| Under 75 years of age | 66.9 | 77.9 |
| Over 75 years of age | 59.5 | 73.1 |
| Whole population | 63.7 | 76.0 |

**Table 8. Fluad vs Fluzone, Frey, Poland, Vaccine 2003, Adults 18-64**

| H1 | GMT day 28-1 | GMT day 28-2 | Med req'd Yr 1 | Med req'd Yr 2 |
|---|---|---|---|---|
| Fluad | 951 | 216 | 39% | 35% |
| Fluzone | 850 | 263 | 26% | 24% |

**Table 9. Elderly, Treanor, JID 2006**

| H1Bv | Pre | Post | %SC |
|---|---|---|---|
| Std VX | 15 | 38 | 21 |
| 15µg | 14 | 25 | 12 |
| 45µg | 18 | 42 | 26 |
| 135µg | 17 | 38 | 20 |

In general, the elderly are a difficult population for influenza vaccination. While increasing the amount of vaccine administered provides some benefit, it is not a proportional increase. Adjuvants may also help increase immunogenicity but they also increase the possibility of adverse reactions. Thus, there has been limited improvement in boosting the immune response in elderly to influenza by increasing the amount of HA administered. There is still a need for improved vaccine formulations for the elderly which provide increased immunity for the elderly.

### Summary of the Invention

The present invention is defined by the claims.

The invention is generally directed to methods of stimulating immune responses in older and elderly humans. The present invention includes compositions and methods of stimulating an immune response in a human, comprising the step of administering to the human a composition comprising at least a portion of at least one flagellin and at least a portion of at least one antigen, wherein the composition is administered to the human in a dose sufficient to provoke an immune response where the human is an elderly human, that is a human who is older than 49 years of age. In certain instances the compositions are administered to humans who are older than 55 years of age, or older than 60 years of age or older than 65 years of age or older than 70 years of age or older than 75 years of age or older than 80 years of age.

The compositions of the present disclosure comprising the invention comprise at least a portion of at least one flagellin and at least a portion of at least one antigen. The flagellin and the antigen may be associated in different ways. For example, the flagellin and the antigen may be part of a fusion protein in which the flagellin portion is associated with the antigen by constructing a DNA or RNA sequence that when expressed produces a protein containing both flagellin and antigen portions as part of a protein. The flagellin portion and antigen portion can be associated in other ways, for example each of these portions may be associated with a particle or some type of carrier where both the flagellin portion and the antigen portion may be attached to the carrier or particle.

The present disclosure comprising the invention also relates to compositions for use in stimulating an immune response in an elderly human population, comprising the step of administering to the human population a composition that includes a fusion protein comprising at least a portion of at least one flagellin and at least a portion of at least one antigen, wherein seroconversion rate is at least 50% or at least 60% or at least 70% or at least 80% or at least 90% or at least 95% or at least 99%

The present disclosure comprising the invention also relates to compositions for use in stimulating an immune response in an elderly human population, comprising the step of administering to the human population a composition that includes a fusion protein comprising at least a portion of at least one flagellin and at least a portion of at least one antigen, wherein the seroresponse rate is at least50% or at least 60% or at least 70% or at least 80% or at least 90% or at least 95% or at least 99%

The present disclosure comprising the invention also relates to compositions for use in stimulating an immune response in an elderly human population, comprising the step of administering to the human population a composition that includes a fusion protein comprising at least a portion of at least one flagellin and at least a portion of at least one antigen, wherein the seroprotection rate is at least 50% or at least 60% or at least 70% or at least 80% or at least 90% or at least 95% or at least 99%.

### Figures

Figure 1 - Amino Acid Sequence of STF2.HA1 (VAX125)
Figure 2A and B - Graphic representation of peak GM HAI response measured 14 days after vaccination with VAX125 among subjects ≥ 65 years old (Figure 2A). GM HAI titers measured from day 0 to 28 after a single dose of VAX125 ranging from 0.5 to 8 µg among subjects ≥ 65 years old (Figure 2B)
Figure 3 - Plasmid map for Vax 128A, Vax 128B and Vax 128C
Figure 4 - Nucleotide sequence for Vax 128A (HL184 STF2.HA1-2 CA7 (CA07 H1N1))
Figure 5 - Amino acid sequence for Vax 128A (HL184 STF2.HA1-2 CA7 (CA07 H1N1))
Figure 6 - Nucleotide sequence for Vax 128B (HL185 STF2.HA1-2 CA7 (CA07 H1N1))
Figure 7 - Amino acid sequence for Vax 128B (HL185 STF2.HA1-2 CA7 (CA07 H1N1))
Figure 8 - Nucleotide sequence for Vax 128C (HL186 STF2.HA1-2 CA7 (CA07 H1N1))
Figure 9 -Amino acid sequence for Vax 128C (HL186 STF2.HA1-2 CA7 (CA07 H1N1))

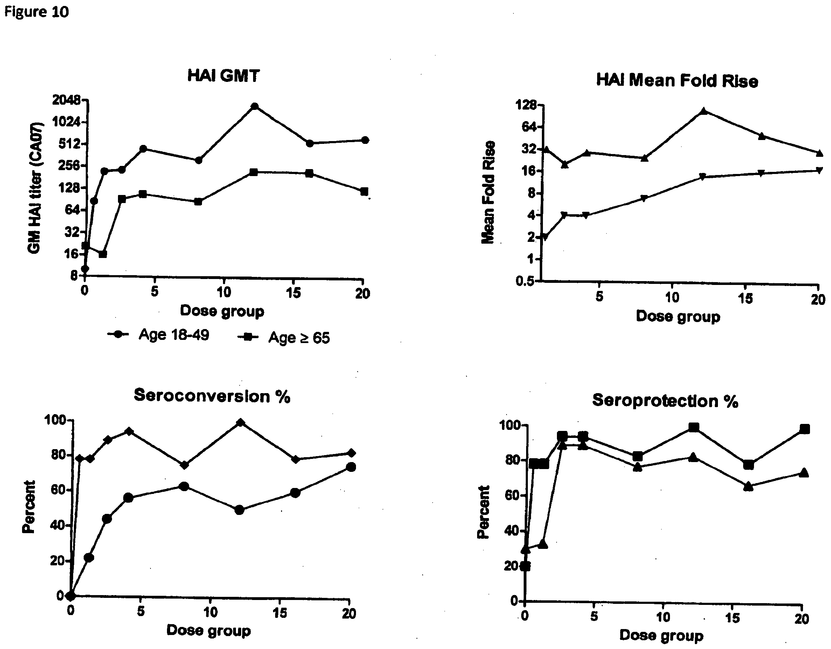

Figure 10 - Comparison of immune response by dose among 112 subjects 18-49 years old and 100 subjects ≥ 65 years old who received a single IM dose of VAX128 (CA07). Note: responses to VAX128A-C have been pooled

### Detailed Description of the Invention

The features and other details of the invention, either as steps of the invention or as combinations of the parts of the invention will now be more particularly described, and pointed out in the claims.

In an instance the disclosure is a method of stimulating an immune response in a human, comprising the step of administering to the human a composition comprising at least one portion of at least one antigen and at least one portion of at least one flagellin, wherein the composition is administered to a human that is at least 49 years old. Administration of the composition to the human can provide protective immunity against an infection consequent to exposure of the human to a source of the antigen.

The compositions of the present dislcosure comprising the invention comprise at least a portion of at least one flagellin and at least a portion of at least one antigen. The flagellin and the antigen may be associated in different ways. For example, the flagellin and the antigen may be part of a fusion protein in which the flagellin portion is associated with the antigen by constructing a DNA or RNA sequence that when expressed produces a protein containing both flagellin and antigen portions as part of a protein. The flagellin portion and antigen portion can be associated in other ways, for example each of these portions may be associated with a particle or a carrier where both the flagellin portion and the antigen portion, including but not limited to proteins, lipoproteins, carbohydrates, polysaccharides and/or lipids, may be attached to the carrier or particle. The particle or carrier can be any vehicle by which the flagellin portion and the antigen portion can be associated such that when introduced to a human an immune response is generated. Particle formulations may be made of a variety of materials, including but not limited to lipids, proteins, waxes or amino acids, polysaccharides, polyacrylic substances or organic acids. In one embodiment, the flagellin portion and antigen portion may be associated with virosomes as, for example, described in US Patent Publication No. 20100136053. Other potential particles to which the antigen(s) and flagellin(s) may be attached may include but are not limited to virus-like particles, biodegradable microspheres, liposomes, peptide nanoparticles, nanoparticles and self-assembling virus like particles as described in WO2010/002818. In one embodiment flagellin and antigen are associated with self-assembling peptide nanoparticles (SAPN). SAPN are described in PCT patent application WO 2009/109428. These nanoparticles are comprised of aggregates of a continuous peptidic chain comprising two oligomerization domains connected by a linker segment wherein one or both oligomerization domains incorporate T-cell epitopes and/or B-cell epitopes within their peptide sequence.

Antigens that can be used in combination with flagellin in the compositions are any antigen that will provoke an immune response in a human. Antigens used in the compositions of the present disclosure invention include viral antigens such as influenza viral antigens (e.g. hemagglutinin (HA) protein, matrix 2 (M2) protein, neuraminidase), respiratory synctial virus (RSV) antigens (e.g. fusion protein, attachment glycoprotein), papillomaviral (e.g. human papilloma virus (HPV), such as an E6 protein, E7 protein, L1 protein and L2 protein), Herpes Simplex, rabies virus and flavivirus viral antigens (e.g. Dengue viral antigens, West Nile viral antigens), hepatitis viral antigens including antigens from HBV and HC. Antigens used in the compositions of the present invention include bacterial antigens including those from *Streptococcus pneumonia, Haemophilus influenza, Staphylococcus aureus, Clostridium difficile* and enteric gram-negative pathogens including *Escherichia, Salmonella, Shigella*, *Yersinia, Klebsiella, Pseudomonas*,*Enterobacter*, *Serratia*, *Proteus.* Antigens used in the compositions of the present disclosure include fungal antigens including those from *Candida spp., Aspergillus spp., Crytococcus neoformans, Coccidiodes spp., Histoplasma capsulatum, Pneumocystis carinii, Paracoccidiodes brasiliensis, Plasmodium folciparum, Plasmodium vivax, Plasmodium ovale, and Plasmodium malariae.*

The ratio of flagellin to antigen in the compositions of the present invention may be from about 100:1 to about 1:100. In other instances the ratio of flagellin to antigen may be from about 1:20 to about 20:1. In a preferred instance of flagellin and influenza antigen the ratio of flagellin to influenza antigen is from about 1:20 to about 1:5. In some instances especially those where flagellin and influenza antigen are linked in a fusion protein the ratio of flagellin to influenza antigen may be from 5:1 to 1:5.

The compositions of the present disclosure may include one or more types of flagellin associated with one or more type of antigens. With respect to particle carriers several different flagellin may be associated with one or more antigens which may be on the same or different particles.

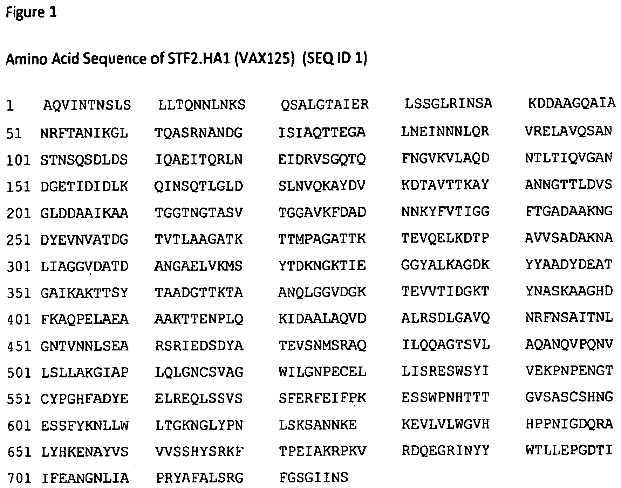

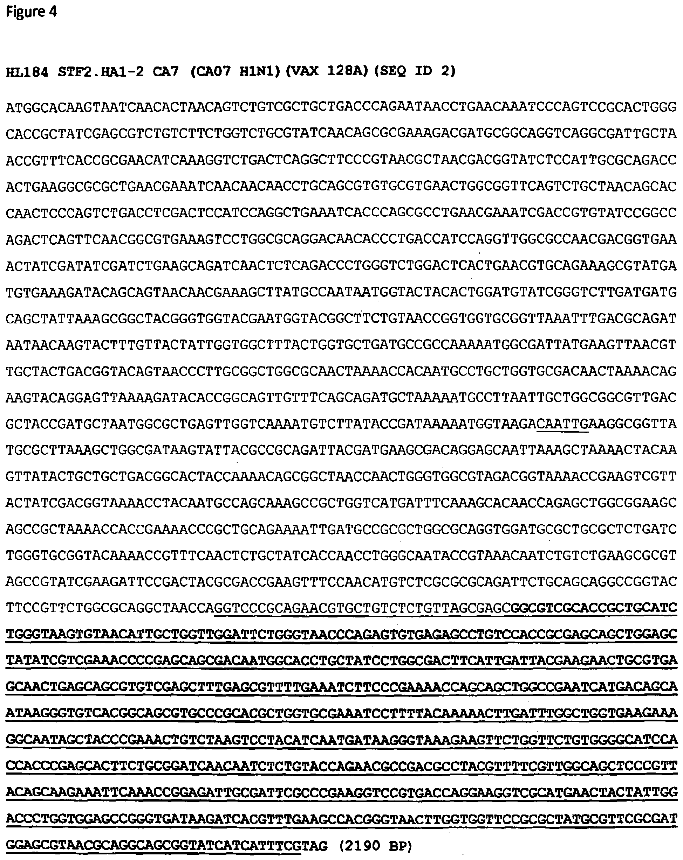

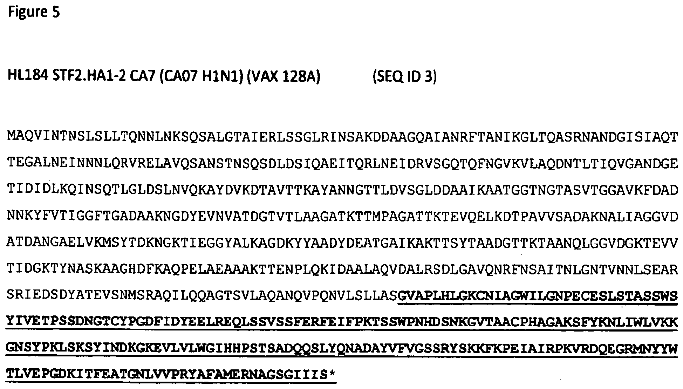

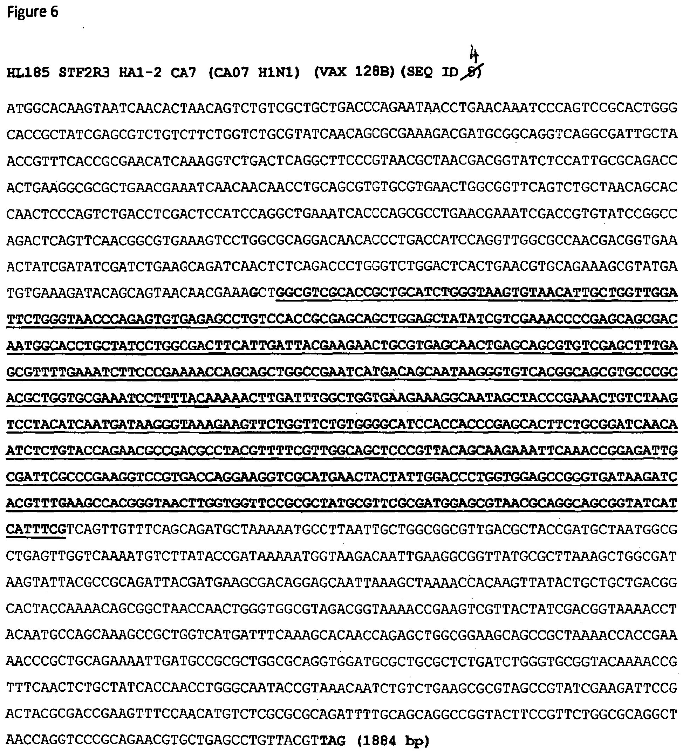

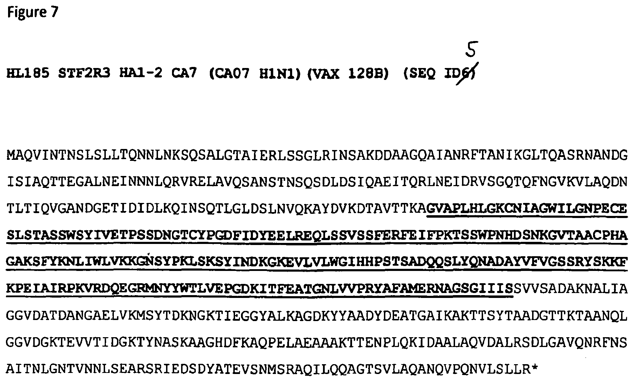

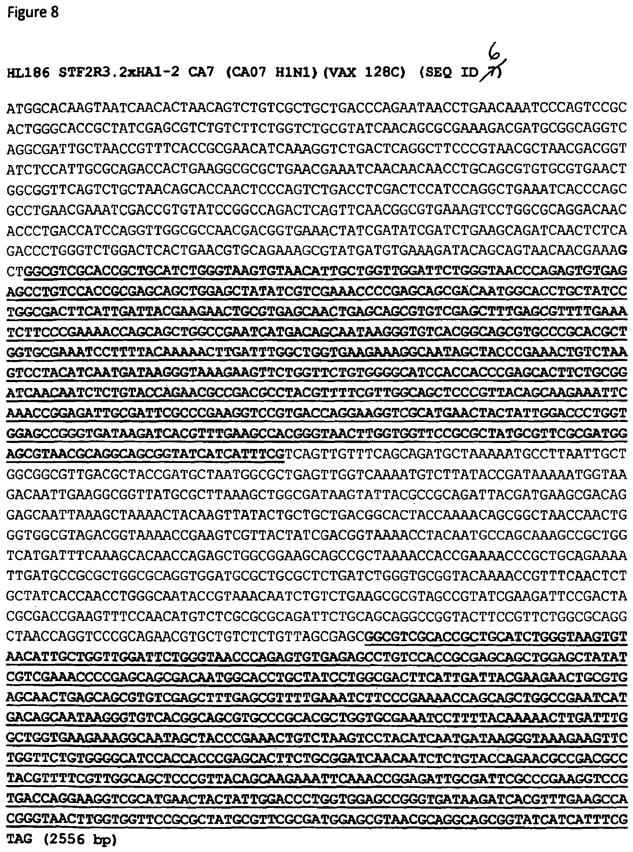

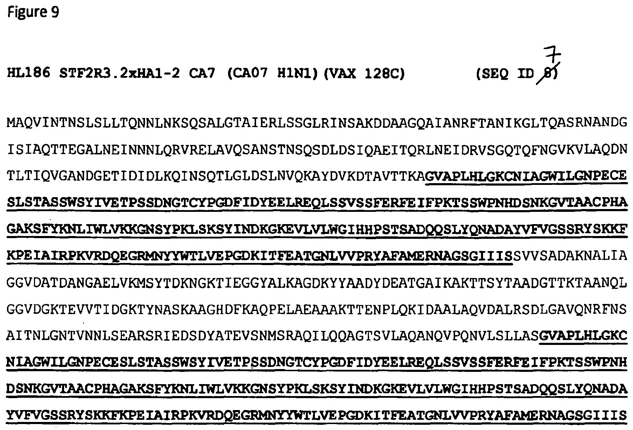

The antigen contained within the compositions of the present invention is an antigen from influenza virus. A preferred antigen is hemagglutinin (HA). In preferred instances the HA sequences are conjugated to flagellin or to engineered flagellins as described in WO 2009/128950. Preferred instances of the present invention that utilize HA as an antigen are VAX 125, VAX 128A, VAX 128B and VAX 128C. VAX125, also known as STF2.HA1(SI), is a recombinant fusion protein that consists of Salmonella typhimurium flagellin type 2 (STF2), a TLR5 ligand, fused at its C-terminus to the globular head (amino acids 62-284) of the HA1 domain of the HA of influenza A/Solomon Islands/3/2006 (H1N1) and has a molecular mass of 77,539 kDa. By molecular weight approximately two-thirds of the vaccine composition is flagellin and one-third is influenza HA globular head. Thus, a 3 ug dose consists of 2 ug of flagellin and 1 ug of influenza HA. The amino acid sequence of VAX 125 (SEQ ID 1) is shown in Figure 1. VAX128A, also known as HL184 STF2.HA1-2 CA7, is a recombinant fusion protein that consists of Salmonella typhimurium flagellin type 2 (STF2), a TLR5 ligand, fused at its C-terminus to the globular head of the HA1 domain of the HA of Influenza A/California/07/2009 (H1N1) a pandemic strain of influenza. The nucleotide (SEQ ID 2) and amino acid (SEQ ID 3) sequences of VAX 128A are shown in Figures 4 and 5, respectively. VAX128B, also known as HL185 STF2.HA1-2 CA7, is an R3 fusion where the D3 domain of the flagellin has been removed and replaced with the globular head of the HA1 domain of the HA of influenza A/California/07/2009 (H1N1). The nucleotide (SEQ ID 4) and amino acid (SEQ ID 5) sequences of VAX 128B are shown in Figures 6 and 7, respectively. VAX128C, also known as HL186 STF.2.HA1-2 CA 7, is a R32x fusion wherein the D3 domain of the flagellin has been removed and replaced with the globular head of the HA1 domain of the HA of Influenza A/California/07/2009 (H1N1) and the C-terminus of this flagellin is fused to the globular head of the HA1 domain of the HA of Influenza A/California/07/2009 (H1N1). Thus, the fusion protein contains two copies of the HA antigen per flagellin unit. The nucleotide (SEQ ID 6) and amino acid (SEQ ID 7) sequences of VAX 128B are shown in Figures 8 and 9, respectively. In VAX128C there are two copies of the HA globular head and the proportion of HA in the vaccine composition increases to 54% from 33% in the VAX128A construct.

Advantages of Applicant's claimed methods include the ability to generate a protective immune response in an elderly population employing relatively low doses of antigens to infectious agents (e.g. influenza). The elderly are typically at high risk for disease consequent to exposure to an infectious agent, such as an influenza antigen, due, in part, to their inability to mount a suitable and sustainable immune response to an infectious antigen. A proposed approach to increase an elderly human's response to an antigen is to increase the dose of antigen and the frequency of dosing. However, such alternative treatment protocols have resulted in less than optimal immune responses to antigens and diminished the ability to provide protective immunity to disease consequent to exposure to antigens in elderly populations (See the tables herein provided concerning prior art products and studies). The methods employed herein improve immune responses in elderly populations so that an excess of 40% of the elderly population mount a suitable and sustainable immune response to an antigen administered in a relatively low dose, which also has the advantage of minimal side effects. The compositions of the present invention are more immunogenic and less reactogenic than prior art vaccines.

In one instance the human to be treated with the compositions and methods of the present invention is older than 65 years old, or older than 70 years of age or older than 75 years of age or older than 80 years of age. The dose of compositions of the present invention may be selected to optimize the effects in the elderly while seeking to reduce any untoward side-effect. For example in the fusion protein compositions comprising flagellin and HA (as described in the case of VAX 125, VAX 128A, VAX 128B and VAX 128C) the does may be selected to optimize the immunogenic response while attempting to keep reactogenicity low. At least one dose selected from the group consisting of a 0.1µg, 0.5µg, 1µg dose, 2µg dose, 3µg dose, 4µg dose, 5µg dose, 6µg dose, 7µg dose, 8µg dose, 9µg dose, 10µg dose, 15µg dose, 20µg dose, 25µg dose and a 30µg dose may be sufficient to induce an immune response in elderly humans. The dose of the fusion protein may be administered to the human within a range of doses including from about 0.1µg to about 500µg, 1µg to about 100µg, 1µg to about 50µg, from about 1µg to about 30µg, from about 1µg to about 25µg, from about 1µg to about 20µg, from about 1µg to about 15µg, from about 1µg to about 10µg, from about 2µg to about 50µg, 2µg to about 30µg, from about 2µg to about 20µg, from about 2µg to about 10µg, from about 2µg to about 8µg, from about 3µg to about 50µg, 3µg to about 30µg, from about 3µg to about 20µg, from about 3µg to about 10µg, from about 3µg to about 8µg, from about 3µg to about 5µg, from about 4µg to about 50µg, 4µg to about 30µg, from about 4µg to about 20µg, from about 4µg to about 10µg, from about 4µg to about 8µg, from about 5µg to about 50µg, 5µg to about 30µg, from about 5µg to about 20µg, from about 5µg to about 10µg, from about 5µg to about 9µg, and from about 5µg to about 8µg. With respect to fusion protein compositions comprising flagellin and influenza antigen the dosage refers to the amount of protein present in the vaccine given to the human. Some of the protein quantity relates to the antigen and some of the protein quantity relates to the flagellin. For example, in VAX 125 and VAX 128A and VAX128B the HA1 antigen comprises about 33% of the molecular weight of the construct. In VAX128C there are two copies of the HA globular head and the proportion of HA in the vaccine increases to 54% from 33% in the VAX128A construct. In instances that use antigens other than HA it may be more useful to characterize the amount of the dosage by the amount of antigen, especially of the antigen is of a non-proteinaceous origin. In any event, the flagellin/antigen compositions of the present invention provide superior properties to the prior art compositions in treatment of the elderly.

With respect to compositions in which the flagellin and antigen are delivered via particles the dosage will depend on the type of particle used and the ratio of flagellin to antigen present in the composition. With respect to compositions in which flagellin and the antigen are present in ratios other than 1:1 the dosage will depend on the ratio of components. With respect to antigens other than influenza antigens the dosage will depend on the antigen used and the ratio of flagellin to the antigen. One of skill in the art may readily be able to determine the optimal dosing of the flagellin compositions based on the carrier, antigen and ratio of flagellin to antigen.

The antigen component of the fusion protein compositions of the present invention are influenza antigens (influenza A, influenza B, influenza C antigen). In preferred instances of the compositions at least one member selected from the group consisting of a hemagglutinin influenza antigen (e.g., the fusion protein includes SEQ ID NO. 1 (Figure 1)), a matrix 2 (M2, such as an extodomain of M2, also referred to as "M2e") or a neuraminidase influenza antigen. The influenza antigen can include an H1N1 influenza antigen. In preferred instances the antigen is fused to a flagellin molecule sequence such as described in WO 2009/128950. The compositions of the present invention may comprise one or more fusion proteins with one or more antigen sequences included in each fusion protein.

The composition can be administered intramuscularly to the human in a single or in multiple doses. The method can further include the step of administering at least one subsequent dose of the flagellin/antigen composition to the human.

The immunogenic compositions for use according to the present invention may be delivered as a standard 0.5 ml injectable dose and contain from about 0.1µg to about 50µg of antigen. In a preferred embodiment of the immunogenic compositions for use according to the present invention is a standard 0.5 ml injectable dose and contains from about 3µg to about 20µg of antigen. The vaccine volume may be between 0.25 and 1.0 ml, suitably between 0.5 ml and 1.0 ml, in particular a standard 0.5ml. A vaccine dose according to the present invention may be provided in a smaller volume than conventional dosing. Low volume doses according to the present invention are suitably below 0.5ml, typically below 0.3ml and usually not less than 0.1 ml.

Accordingly, in one aspect of the disclosure it is provided for a composition, method or use as claimed herein wherein the immune response produced by administration of the composition in a human population where the humans are about 49 years old to about 64 years old or where the humans are older than 55 years of age, or older than 60 years of age or older than 65 years of age or older than 70 years of age or older than 75 years of age or older than 80 years of age and has a seroconversion rate of greater than 50%, greater than 60%, greater than 65% greater than 70%, greater than 75%, greater than 80%, greater than 85%, greater than 90%, greater than 95% or greater than 99%.

Accordingly, in one aspect of the disclosure it is provided for a composition, method or use as claimed herein wherein the immune response produced by administration of the composition in a human population where the humans are about 49 years old to about 64 years old or where the humans are older than 55 years of age, or older than 60 years of age or older than 65 years of age or older than 70 years of age or older than 75 years of age or older than 80 years of age and has a seroprotection rate of greater than 50%, greater than 60%, greater than 65%, greater than 70%, greater than 75%, greater than 80%, greater than 85%, greater than 90%, greater than 95% or greater than 99%.

Seroresponsive means an increase in HAI antibody titer of at least fourfold with a minimum post vaccination titer of 40.

Seroprotection means achievement of minimum post vaccination HAI titer of 40 among subjects with prevaccination titers of <40.

Seroconversion rate for anti-HA antibody response is defined as the proportion of subjects in each group having protective post-vaccination titer ≥ 1:40. The seroprotection rate is the percentage of subjects who have an HAI titer before vaccination of < 1:10 and ≥ 1:40 after vaccination. However, if the initial titer is ≥ 1:10 then there needs to be at least a fourfold increase in the amount of antibody after vaccination.

In another aspect of the disclosure it is provided for a composition, method or use as claimed herein wherein the immune response produced by administration of the compositions of the present invention induces functional (HAI) antibodies in the majority of elderly recipients in a dose dependent fashion. In certain instances the composition will induce a neutralizing antibody response of greater than a titer about 50 after 7 days or after 14 days or after 28 days. In other instances the composition will induce a neutralizing antibody response of greater than about a titer of 100 after 7 days or after 14 days or after 28 days. In other instances the composition will induce a neutralizing antibody response of greater than a titer of about 150 after 7 days or after 14 days or after 28 days. In other instances the composition will induce a neutralizing antibody response of greater than about 200 after 7 days or after 14 days or after 28 days.

Accordingly, in one aspect of the disclosure comprising the invention it is provided for a composition, method or use as claimed herein wherein the immune response produced by administration of the composition in an elderly population meets or exceeds one of the following criteria:
- a seroconversion rate of greater than 50%, greater than 60%, greater than 70%, greater than 80%, greater than 90%, greater than 95% or greater than 99%.
- a seroprotection rate of greater than 50%, greater than 60%, greater than 70%, greater than 80%, greater than 90%, greater than 95% or greater than 99%.
- an average of four-fold or greater increase in neutralizing antibody titer at post vaccination.

An "effective amount" when referring to the amount of a composition and a fusion protein administered to the human, refers to that amount or dose of the composition that, when administered to the subject is an amount sufficient for therapeutic efficacy (e.g., an amount sufficient to stimulate an immune response in a subject, an amount sufficient to provide protective immunity in the subject).

The methods of the present invention can be accomplished by the administration of the compositions and fusion proteins of the invention by enteral or parenteral means. Specifically, the route of administration is by intramuscular injection of the composition and fusion protein. Other routes of administration are also encompassed by the present invention including intravenous, intradermal, interaarterial, interperitoneal, intranasal, transdermal, suppositories or subcutaneous routes.

The compositions that include the fusion proteins can be administered alone or as admixtures with conventional excipients, for example, pharmaceutically, or physiologically, acceptable organic, or inorganic carrier substances suitable for enteral or parenteral application which do not deleteriously react with the composition. Suitable pharmaceutically acceptable carriers inclue water, salt solutions (such as Ringer's solution), alcohols, oils, gelatins and carbohydrates such as lactose, amylose or starch, fatty acid esters, hydroxymethylcellulose, and polyvinyl pyrolidine. Such preparations can be sterilized and, if desired, mixed with auxiliary agents such as lubricants, preservatives, stabilizers, wetting agents, emulsifiers, salts for influencing osmotic pressure, buffers, coloring and/or aromatic substances and the like which do not deleteriously react with the compositions administered to the human. Preferred diluents for diluting the vaccines of the present invention include but are not limited to 150mM NaCl with histidine and trehalose.

The compositions, fusion proteins and proteins of the invention can be administered to a subject on a support that presents the compositions, proteins and fusion proteins of the invention to the immune system of the subject to generate an immune response in the subject. The presentation of the compositions, proteins and fusion proteins of the invention would preferably include exposure of antigenic portions of the fusion protein to generate antibodies. The support is biocompatible. "Biocompatible" as used herein, means that the support does not generate an immune response in the subject (e.g., the production of antibodies).

The dosage and frequency (single or multiple doses) administered to a subject can vary depending upon a variety of factors, including, for example, prior exposure to an infection consequent to exposure to the antigen: health, body weight, body mass index, and diet of the subject or health-related problems. Other therapeutic regimens or agents can be used in conjunction with the methods and compositions, proteins or polypeptides of the present invention.

The composition can be administered to the human in a single dose or in multiple doses, such as at least two doses. When multiple doses are administered to the subject, a second or third dose can be administered days (e.g., 1, 2, 3, 4, 5, 6, 7), weeks (e.g., 1, 2, 3, 4, 5, 6, 7, 8, 9, 10), months (e.g., 1, 2, 3, 4, 5, 6, 7, 8, 9, 10) or years (e.g., 1, 2, 3, 4, 5, 6, 7, 8, 9, 10) after the initial dose. For example, a second dose of the composition can be administered about 7 days, about 14 days or about 28 days following administration of a first dose of the composition that includes the fusion protein.

Ranges may be expressed herein as from about one particular value, and/or to about another particular value. When such a range is expressed, another aspect includes from the one particular value and/or to the other particular value. Similarly, when values are expressed as approximations, by use of the antecedent about, it will be understood that the particular value forms another aspect. It will be further understood that the endpoints of each of the ranges are significant both in relation to the other endpoint, and independently of the other endpoint.

The dose of fusion protein is made in reference to microgram doses. The invention is generally directed to treating elderly populations of human to stimulate immune responses to antigens, in particular a protective immune response, employing relatively low doses of the antigenic composition that include the antigen. A description of example of the invention follows.

### Examples

### Example 1

### Phase II, Open-Label, Escalating Dose-Ranging Study

STF2.HA1(SI) (VAX125) is a recombinant fusion protein that consists of *Salmonella typhimurium* flagellin type 2 (STF2), a TLR5 ligand, fused at its C-terminus to the globular head (amino acids 62-284) of the HA1 domain of the HA of influenza A/Solomon Islands/3/2006 (H1N1) and has a molecular mass of 77,539 kDa (SEQ ID 1). Vaccine was supplied in glass vials at either 20 µg/mL or 2 µg/mL, and diluted in dilution buffer (150mM NaCl with histidine and trehalose) to the final concentration on the day of administration. Study materials were prepared by non-blinded pharmacists and provided to blinded clinical staff. Vaccine was administered at a final volume of 0.5 mL by deep intramuscular injection in the deltoid muscle.

A total of 120 community-living adults who were ≥ 65 years of age (the mean age was 72 with a range of 64-84) enrolled across the six dose groups. Subjects were healthy as ascertained by medical history, screening physical examination and screening laboratory analysis. Subjects with active medical conditions, abnormal screening laboratory tests (CBC and differential, and AST and ALT), known allergies to vaccine components, influenza vaccination within the previous 6 months, or receipt of other vaccines within 30 days were excluded from participation.

The study was conducted in an open-label, dose escalation design. Twenty subjects were enrolled in each dose group at up to 3 study sites. Subjects received a single dose at dose levels of 0.5 µg, 1.0 µg, 2.0 µg, 3.0 µg, 5 µg, and 8 µg (Table 1). Subjects were evaluated in the clinic following vaccination at 30 minutes, 4 hours, Day 1, and by phone interview at Day 3. Subjects enrolled in the 5 and 8 µg groups remained under observation at the study site for four hours after vaccination. Subjects were further evaluated during clinic visits on study days 7, 14, and 28 following vaccination. Safety follow-up by telephone contact also occurred at 6 and 12 months after vaccination.

**Table 1. Demographic and Baseline characteristics**

| | | | VAX125 dose (µg) | | | | |
|---|---|---|---|---|---|---|---|
| | 0.5 | 1 | 2 | 3 | 5 | 8 | Total |
| | n=20 | n=20 | n=20 | n=20 | n=20 | n=20 | n=120 |
| Male (%) | 10 (50) | 7 (35) | 10 (50) | 5 (25) | 12 (60) | 9(45) | 54(44) |
| Age (mean) | 72.8 | 71.2 | 71.7 | 71.9 | 70.7 | 70.6 | 71.5 |
| Age (range) | 65-84 | 65-81 | 65-79 | 67-81 | 65-77 | 65-80 | 65-84 |
| Race--White | 20 (100) | 20(100) | 18 (90) | 20 (100) | 19 (95) | 20 (100) | 117 (98) |
| -- Black | 0(0) | 0(0) | 1 (5) | 0(0) | 1(5) | 0(0) | 2 (2) |
| -- Asian | 1 (0) | 1 (0) | 1 (5) | 0(0) | 0(0) | 0(0) | 1 (1) |

### Immunogenicity

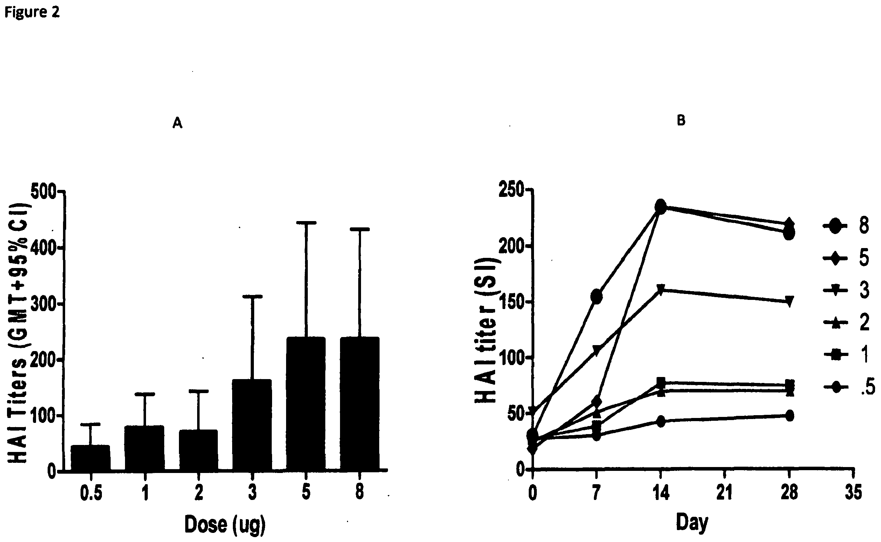

The ability of antibody generated in response to E. *coli-derived* hemagglutinin antigen to inhibit hemagglutination by egg-grown influenza virus is shown in Tables 2 and 3. The geometric mean titer (GMT) of HAI antibody on day 28 after a single dose increased in a dose dependent manner, with the highest post-vaccination GMT seen in subjects who received 5 µg (Table 2). Four-fold or greater antibody responses were seen in the majority of subjects who received doses of 5 or 8 µg. Among subjects who received doses of 5 or 8 µg, a total of 30 (75%) of 40 subjects had four-fold or greater serum HAI antibody responses. In the 5 and 8 µg dose groups 18 (45%) subjects had titers of 1:40 or greater at the beginning of the study before vaccination. Among the 22 subjects with pre-vaccination serum HAI antibody of 1:40 or less, 21 (98%) achieved a titer of at least 1:40 by day 28 after vaccination (Table 3). The kinetics of the antibody titers by group in this study are shown in Figure 2.

The anti-flagellin response as assessed by IgG ELISA appears in Table 4. Levels of anti-flagellin IgG were low in these subjects prior to vaccination. VAX125 induced substantial increases in anti-flagellin antibodies. There was no apparent relationship between the level of anti-flagellin antibody detected prior to vaccination and either the frequency or severity of side effects, or the serum antibody response to the influenza hemagglutinin.

The immune response generated by VAX125 was compared to the H1N1 component of the standard (15 µg) and high dose (60 µg) Fluzone as reported by Falsey et al. (Table 5). The GMT increase in subjects 65 years and older after standard Fluzone increases by about 2-fold and after the high dose by about 4-fold. In contrast the GMT increase in subjects 65 years and older after VAX125 the 5 µg dose increases by more than 10-fold. This translates to higher seroconversion rates and seroprotection rates in the VAX125 groups.

**Table 2. Geometric mean (GMT) HAI antibody titers, mean fold response (MFR) among 120 subjects ≥ 65 years old who received a single dose of VAX125**

| | Dose (µg) | | | | | | | | | | | |
|---|---|---|---|---|---|---|---|---|---|---|---|---|
| | 0.5 | | 1 | | 2 | | 3 | | 5 | | 8 | |
| Day | n=20 | 95% Cl | n=20 | 95% Cl | n=20 | 95% Cl | n=20 | 95% Cl | n=20 | 95% Cl | n=20 | 95% Cl |
| 0 | 27 | 14-52 | 27 | 15-50 | 25 | 12- | 51 | 29-89 | 18 | 10-32 | 3 | 106- 58 |
| 7 | 30 | 16-58 | 39 | 21-70 | 51 | 25- | 106 | 50-225 | 61 | 34-107 | 155 | 86-279 |
| 14 | 43 | 22-83 | 77 | 43-138 | 70 | 34-143 | 160 | 82-311 | 226 | 121-420 | 234 | 127- 431 |
| 28 | 48 | 24- 95 | 75 | 44- 126 | 70 | 34- 143 | 149 | 79-283 | 219 | 115- 413 | 211 | 120-370 |
| MFR | | | | | | | | | | | | |
| 14 | 1.6 | 1-2.5 | 2.8 | 1.6-5.1 | 2.8 | 1.6-4.9 | 3.1 | 2.1-4.8 | 12.6 | 6-25 | 7.7 | 4- 16 |
| 28 | 1.7 | 1-2.9 | 2.7 | 1.6-4.7 | 2.8 | 1.6-4.9 | 2.9 | 1.9-4.6 | 11.7 | 6-25 | 7 | 4- 14 |

**Table 3. Seroconversion (SC) and seroprotection (SP) rates among 120 subjects ≥ 65 years old who received a single dose of VAX125**

| | Dose (ug) of VAX125 (STF2.HA1 (SI)) | | | | | |
|---|---|---|---|---|---|---|
| | 0.5 n=20 | 1 n=20 | 2 n=20 | 3 n=20 | 5 n=20 | 8 n=20 |
| Seroconversion | | | | | | |
| day 14 (%) | 3 (15) | 8(40) | 8 (40) | 8 (40) | 16(80) | 13 (65) |
| day 28 (%) | 3 (15) | 7 (35) | 6 (30) | 7 (35) | 16(80) | 14 (70) |
| Seroprotection | | | | | | |
| day 0 (%) | 10 (50) | 11 (55) | 7 (35) | 15 (75) | 7 (35) | 11 (55) |
| day 14 (%) | 13 (65) | 17 (85) | 14 (70) | 19 (95) | 19 (95) | 20 (100) |
| day 28 (%) | 14(70) | 18(90) | 13 (65) | 19(95) | 19 (95) | 20 (100) |

| | | | | | | |
|---|---|---|---|---|---|---|
| Seroconversion (increase in HI titer of at least 4 fold with a min titer of 40 Seroprotection (min post-vac. titer of 1:40), | | | | | | |

**Table 4. Geometric mean anti-flagellin serum IgG titers (ug/ml) after single dose of VAX125 in adults a 65 years old**

| | Dose (ug/ml) | | | | | |
|---|---|---|---|---|---|---|
| | 0.5 | 1 | 2 | 3 | 5 | 8 |
| Day post vaccination | n=20 | n=20 | n=20 | n=20 | N=20 | n=20 |
| 0 | 5 | 2 | 4 | 2 | 3 | 2 |
| 7 | 8 | 8 | 39 | 17 | 41 | 34 |
| 14 | 19 | 26 | 103 | 49 | 173 | 93 |
| 28 | 16 | 21 | 72 | 35 | 125 | 58 |
| Mean Fold response | | | | | | |
| day 14 | 4 | 14 | 24 | 25 | 55 | 49 |
| day 28 | 3 | 11 | 16 | 18 | 39 | 31 |

**Table 5. Comparative seroresponse against H1N1 antigens in adults over 65 for VAX125 and Fluzone standard dose and high dose**

| | VAX125 dose (ug) | | | Fluzone^{®} dose (ug) | |
|---|---|---|---|---|---|
| | 3 | 5 | 8 | 15 | 60 |
| Number of subjects | 20 | 20 | 20 | 1252 | 2543 |
| GMT (A/H1N1) | | | | | |
| Pre-vaccination | 51 | 18 | 30 | 29 | 29 |
| Post-vaccination | 160 | 226 | 234 | 67 | 116 |
| Fold increase | 3.1 | 12.6 | 7.7 | 2.3 | 4.1 |
| Seroconversion (%) | 40 | 80 | 65 | 23 | 49 |
| Seroprotection (%) | 95 | 95 | 100 | 77 | 90 |

| | | | | | |
|---|---|---|---|---|---|
| A/H1N1 Solomon Islands for VAX125 and H1N1 New Caledonia for Fluzone. Fluzone (Sanofi) standard dose 15 ug, HD high dose 60ug each antigen. From Falsey et al. JID 2009;200:172-80 | | | | | |

### Reactogenicity.

Vaccine was well tolerated in all dose groups (Table 6). No side effect was graded as severe (3) or life-threatening (4) and there were serious no adverse events. Moderate arm pain was observed in about 20% of subjects of subjects who received a dose of 2 µg or greater. Systemic adverse events were usually mild and did not appear to be dose-depended.

C-reactive protein (CRP) levels were measured at baseline and on day 1 (Table 7). The mean fold increase in CRP showed a dose-dependence, with a mean increase of less than 5-fold in subjects receiving the two highest doses. At doses of 3 µg or greater 57% of 60 subjects experienced at least a 2- fold increase in CRP. However, the majority (or none) of CRP increases were not associated with local or systemic symptoms of greater than mild severity or for subjects in stage 1 associated with increases in IL-6, IL-8 or IL-10 (data not shown).

**Table 6. Number of subjects over 65 years with symptoms after vaccination with VAX125**

| | Dose (µg) of VAX125 (STF2.HA1 (SI)) | | | | | |
|---|---|---|---|---|---|---|
| | 0.5 | 1 | 2 | 3 | 5 | 8 |
| Local symptoms | n=20 | n=20 | n=20 | n=20 | n=20 | n=20 |
| Grade 0 | 14(70) | 9(45) | 12(60) | 6(30) | 4(20) | 6(30) |
| Grade 1 | 5 | 10(50) | 3(15) | 10(50) | 12(60) | 10(50) |
| Grade 2 | 1 | 1(5) | 5(25) | 4(20) | 4(20) | 4(20) |
| Grade 3 | 0 | 0 | 0 | 0 | 0 | 0 |
| Grade 4 | 0 | 0 | 0 | 0 | 0 | 0 |
| Systemic symptoms | | | | | | |
| Grade 0 | 16(80) | 18(90) | 10(50) | 12(60) | 11(55) | 12(60) |
| Grade 1 | 4(20) | 0 | 6(30) | 4(20) | 6(30) | 7(35) |
| Grade 2 | 0 | 2(10) | 4(20) | 4(20) | 3(15) | 1(5) |
| Grade 3 | 0 | 0 | 0 | 0 | 0 | 0 |
| Grade 4 | 0 | 0 | 0 | 0 | 0 | 0 |

**Table 7. CRP response among adults ≥ 65 years old after one dose of VAX125**

| | Dose (µg) | | | | | |
|---|---|---|---|---|---|---|
| | 0.5 | 1 | 2 | 3 | 5 | 8 |
| No. of subjects | n=20 | n=20 | n=20 | n=20 | n=20 | n=20 |
| GM CRP day 0 | 1.0 | 1.3 | 1.2 | 1.7 | 1.6 | 1.4 |
| GM CRP day 1 | 1.0 | 2.0 | 2.2 | 3.4 | 4.7 | 4.0 |
| Max. CRP day 1 | 4.9 | 11.9 | 13.7 | 13.5 | 22.8 | 33.7 |
| GM CRP fold inc. | 1.1 | 1.6 | 1.9 | 2.0 | 2.9 | 2.8 |
| No. (%) ≥ 2-fold inc. | 1 (5) | 4(20) | 7 (35) | 10(50) | 13 (65) | 11 (55) |

### Discussion

VAX125 was both safe and immunogenic at the dose range tested, but the elderly appear much less sensitive to the VAX125 in terms of immune and cytokine response. The peak GMT HAI titers in the 3µg elderly group was 160 which was similar to the 0.1µg dose in young adults. The peak titers in the 0.3 and 0.5µg dose in the young adults were higher than the elderly (Table 8). There is a four-fold or greater serum HAI responses in 61 of 96 (64%) subjects who received doses of 0.5 µg or greater, including in 46 of 72 subjects who received doses from 0.5 µg to 2 µg. The GM HAI titer after a single 0.5 µg dose of VAX125 was similar to the titer observed in the 5 µg dose in subjects over 65 years, suggesting that it requires nearly a 10-fold increase in dose to obtain the same titer in the elderly.

**Table 8. Geometric mean HAI antibody titers, seroprotection and seroconversion rates for H1N1 Solomon Island among young adult subjects who received one IM dose of VAX125.**

| HAI GMT | | | | | | MFR | | SR | SP |
|---|---|---|---|---|---|---|---|---|---|
| Dose | No. of Subjects | Day 0 | Day 14 | Day 28 | Day 180 | Day 14 | Day 28 | NO. (%) | B < 40 |
| 0.1 | 8 | 87 | 123 | 174 | 293 | 1.4 | 2 | 1(13) | 1/1 |
| 0.3 | 8 | 113 | 293 | 381 | 226 | 2.6 | 3.4 | 4(50) | 3/3 |
| 1 | 8 | 160 | 698 | 905 | 587 | 4.4 | 5.7 | 4(50) | 2/2 |
| 2 | 8 | 95 | 587 | 587 | 226 | 6.2 | 6.2 | 5(63) | 1/1 |
| 3 | 8 | 174 | 987 | 640 | 351 | 5.7 | 3.7 | 3(38) | 1/1 |
| 5 | 8 | 62 | 1522 | 1174 | 359 | 24.7 | 19 | 7(88) | 2/2 |
| 8 | 8 | 40 | 320 | 349 | 147 | 8 | 8.7 | 5(63) | 2/3 |
| Control | 16 | 91 | 87 | 84 | - | 1 | 1 | 0 | 0/2 |
| 0.5 | 24 | 37 | - | 285 | - | - | 7.8 | 16 (67) | 9/10 |
| 1 | 16 | 108 | 494 | 473 | - | 4.6 | 4.4 | 8(50) | 3/4 |
| 2 | 16 | 62 | 795 | 761 | - | 12.9 | 12.3 | 13(81) | 5/5 |

Table 9 is a comparison of the mean and maximum CRP values for healthy subjects older than about 65 years old and subjects 18-49 years old. The geometric means do not vary until you reach the 3µg group where the mean is nearly double in the young adults. The upper 95% confidence limit and the maximum value are both less in the elderly.

**Table 9. Comparison of CRP in elderly and young adults after VAX125.**

| Dose (µg) | 1 | | 2 | | 3 | |
|---|---|---|---|---|---|---|
| Age group | >65 | 18-49 | >65 | 18-49 | >65 | 18-49 |
| No. subjects | 20 | 24 | 20 | 24 | 20 | 8 |
| Geometric mean | 2 | 2.2 | 2.2 | 2.4 | 3.4 | 5.9 |
| Upper 95% Cl | 3.2 | 4.7 | 3.4 | 4.6 | 5 | 19.9 |
| Maximum | 11.9 | 43.7 | 13.7 | 33.2 | 13.5 | 83 |

While these data may suggest that the optimal dose in the elderly is greater than 3µg, doses as low as this still produce an HAI response to the H1N1 antigen that is better than the standard Fluzone TIV and is similar to the response observed with the high dose Fluzone formulation (Table 10).

**Table 10. Immunological response of VAX125 compared to standard dose and high dose Fluzone in elderly subjects 65 years or older.**

| | VAX125 | | Fluzone TIV | |
|---|---|---|---|---|
| Dose of H1N1 | 2 µg | 3 µg | 15 µg | 60 µg |
| No. of subjects | 20 | 20 | 1252 | 2543 |
| SP (%) 70 | 70 | 95 | 77 | 90 |
| SC(%) | 35 | 40 | 23 | 49 |
| GMT HAI Titer | 70 | 149 | 67 | 116 |

**Table 11 Comparative Seroresponse against H1N1 antigens in adults over 65 for VAX 125 and Fluzone standard dose and high dose**

| | VAX 125 dose (µg) | | | Fluzone dose (µg) | |
|---|---|---|---|---|---|
| | 3 | 5 | 8 | 15 | 60 |
| Number of Subjects | 20 | 20 | 20 | 1252 | 2543 |
| GMT (H1N1) | | | | | |
| Prevaccination | 51 | 19 | 30 | 29 | 29 |
| Post vaccination | 160 | 234 | 234 | 67 | 116 |
| Fold Increase | 3.1 | 12.6 | 7.7 | 2.3. | 4.1 |
| Seroconversion (%) | 40 | 80 | 65 | 23 | 49 |
| Seroprotection (%) | 95 | 95 | 100 | 77 | 90 |

**Table 12. Clinical Evaluation of VAX 125 Phase I**

| | | | VAX 125 dose range (µg) | | |
|---|---|---|---|---|---|
| | Timing | Control N=16 | 0.1-0.3 N=16 | 1-3 N=56 | 5-8 N=16 |
| GMT | Day 0 | 91 | 99 | 102 | 50 |
| | Day 14 | 87 | 190 | 672 | 698 |
| | Day 28 | 84 | 258 | 640 | 640 |
| GMT fold increase | At day 14 | 1.0 | 1.9 | 6.6 | 14.1 |
| | At day 28 | 0.9 | 2.6 | 6.2 | 12.9 |
| | Seroresponse* | 0 | 5(31%) | 33(59%) | 12(75%) |
| | Seroprotection | 0 of 2 | 4 of 4 | 12 of 13 (92%) | 5 of 6 (83%) |

| | | | | | |
|---|---|---|---|---|---|
| *Seroresponse: increase in HAI antibody titer of at least fourfold with a minimum post vaccination titer of 40 **Seroprotection: achievement of minimum post vaccination HAI titer of 40 among subjects with prevaccination titers of <40. | | | | | |

VAX125 induces functional (HAI) antibodies in the majority of elderly recipients in a dose dependent fashion. The 5.0 and 8.0 µg doses elicited high HAI GMT (226 and 234, respectively) and high GMT fold response (12.6 and 7.7, respectively) values. The 5.0 µg dose was the most immunogenic. The 5.0 µg and 8.0 µg doses elicited high seroconversion rates (80% and 65%, respectively) and high seroprotection rates (95% and 100%, respectively). The peak GMT values in healthy adults ≥ 65 were similar to those observed with 0.5 µg dose in young adults. This suggests that a high dose formulation is required for the elderly. The ability to produce a high dose influenza vaccine in *E. coli* for the elderly is unique to this product.

### Example 2

### Construction and Expression of Vax 128A, Vax 128B and Vax 128C

### Cloning and Expression

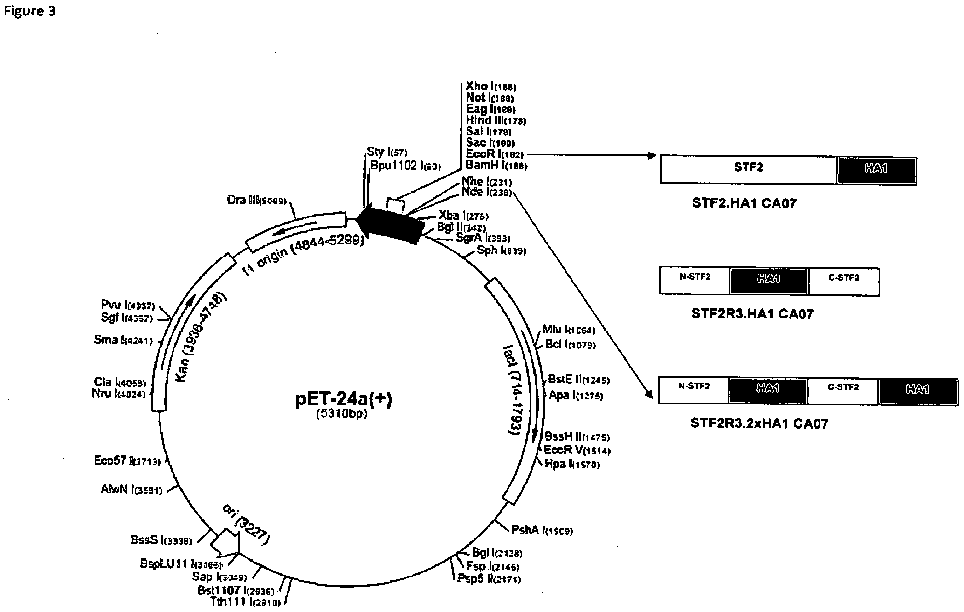

*E. coli* clones producing each of the three vaccine candidates Vax 128A, Vax 128B and Vax 128C were produced in a similar manner, but with differences in plasmid preparation specific to each construct. Plasmids encoding for STF2.HA1 (CA07) and STF2R3.HA1 (CA07) were mutated from plasmids encoding for STF2.HA1 (CA04) and STF2R3.HA1 (CA04). The plasmid encoding for STF2R3.2xHA1 (CA07) was produced from the STF2.HA1 (CA07) and STF2R3.HA1 (CA07) plasmids. Details of the plasmid construction and clone selection follow. A plasmid map for the vaccine candidates is shown in Figure 3.

### Plasmid Construction for Vax 128A (STF2. HA1 (CA07))

A multi step PCR procedure was performed to produce the plasmid encoding for STF2.HA1 (CA07). A plasmid encoding for STF2.HA1 (CA04) was initially produced, and then mutated to code for STF2.HA1 (CA07). To make STF2.HA1 (CAO4), DNA encoding STF2 was fused with DNA encoding the HA1 globular head domain of the (CA04) HA protein. DNA encoding STF2 was amplified from the pET24a-STF2.HA1 FL plasmid. DNA encoding the HA globular head protein was synthesized by an outside vendor (DNA 2.0, Menlo Park, CA) and amplified by PCR. After gel purification of both the SFT2 and HA1 (CA07) DNA, the fragments were fused together. The final PCR product (DNA encoding the STF2.HA1 (CA04) fusion protein) was digested with restriction enzymes Ndel and EcoRI and ligated to the pET24a plasmid. Since there is only one residue difference between the HA portion of STF2.HA1-2 (CA04) and STF2.HA1 (CA07), STF2.HA1 (CA07) was made by site-directed mutagenesis of STF2.HA1 (CA04). The recombinant DNA sequence was confirmed by an outside vendor (Genewiz Inc.).

### Plasmid Construction for Vax 128B (STF2R3. HA1 (CA07))

As with STF2.HA1 (CA07), a plasmid encoding for STF2R3.HA1 CA4 was initially produced and then mutated to code for STF2R3.HA1 (CA07). To make STF2R3.HA1 (CA04), DNA encoding portions of the N and C terminal sections of STF2 was fused with DNA encoding the HA1 globular head domain of the CA4 HA protein. This has the effect of replacing the D3 domain of STF2 with HA1-2 (CA04) in the fusion protein. DNA encoding for the portions of the N and C terminal sections of STF2 was PCR amplified from the pET24a-STF2.HA1 (CA04) plasmid. DNA encoding for HA1 (CA04) was PCR amplified from the same plasmid. Gel purified STF2 and HA1 (CA04) fragments were fused, and the final PCR product was digested with restriction enzymes Ndel and EcoRI and ligated to the pET24a plasmid. Since there is only one residue difference between the HA potion of STF2R3.HA1 (CA04) and STF2R3.HA1 (CA07), STF2R3.HA1 (CA07) was made by site-directed mutagenesis of STF2R3.HA1 (CA04). The recombinant DNA sequence was confirmed by an outside vendor (Genewiz Inc.).

### Plasmid Construction for Vax 128C (STF2R3. 2xHA1 (CA07))

The plasmid encoding for STF2R3.2×HA1 (CA07) was made from a fusion of the DNA from the STF2R3.HA1 (CA07) plasmid and the STF2.HA1 (CA07) plasmid. Both plasmids were digested separately with Ndel and Mfel enzymes. Specific fragments of the STF2R3.HA1 (CA07) (as insert) and STF2.HA1 (CA07) (as vector) were gel purified and ligated to form a complete DNA sequence encoding for STF2R3.2xHA1 (CA07). The common restriction site is in the C terminal section of STF2. The recombinant DNA sequence was confirmed by an outside vendor (Genewiz Inc.).

### Cloning

The DNA was used to transform commercially available *E. coli* BLR(DE3) cells (Novagen, Cat. No. 69053). *E. coli* BLR(DE3) cells from Novagen are a recA derivative of a BL21 strain of *E*. *coli* that improves plasmid monomer yield and may help to stabilize included plasmids.. The cells are *Ion* and *ompT* protease deficient, and are lysogenic for lambda prophage that contains an IPTG inducible T7 RNA polymerase. The resulting BLR(DE3) clones, were used to produce a research cell bank for each vaccine candidate. To generate the banks, a selected clone was grown in LB media, supplemented with 0.5% Glucose, and 25 µg/mL kanamycin. Cells were frozen in 1 mL cryovials at -60 to -80 °C following the addition of glycerol to 7% final concentration. In protein expression studies, proteins corresponding to the expected molecular weights of the vaccine candidates were easily visualized by Coomassie Blue staining and by Western blot analyses using an anti-flagellin monoclonal antibody.

### Protein Production

The constructs described below are all comprised of combinations of *Salmonella fyphimurium fljB* flagellin (STF2) and a portion of the globular head domain of the protective hemagglutinin antigen from the novel H1N1 emerging in 2009 (A/California 07/2009).

### Manufacture of VAX128A

A pET24a plasmid encoding production of STF2.HA1-2 CA07 is used to transform commercially available *E. coli* BLR(DE3) cells. These cells are expanded to manufacture both research and cGMP master cell banks. To produce the target protein, a vial of banked cells was expanded in shake flasks using synthetic media and grown to a specific optical density. A specific amount of cells were then transferred to a bioreactor containing a different synthetic media. The bioreactor was run under automatic control (T=30C, pH = 7, DO >= 30%) in batch mode until the glucose was exhausted. At that point a synthetic feed media is added to the bioreactor at a specific flow rate. After three hours, target protein production was initiated in the culture by addition of IPTG to a concentration of 2 mM. The cells were harvested four hours after induction and the cell paste separated from the conditioned media by centrifugation. After freezing and thawing, the cells were re-suspended in a Tris acetate buffer and lysed by high pressure homogenization. The lysate supernatant was collected after centrifugation. Proteins were precipitated using 14% PEG, and then re-suspended in 6M urea at pH 4. After adjusting the solution pH to 8, the buffer was exchanged into a Tris/acetate buffer by TFF. Triton X-114 and PEG were added and the resulting solution separated into detergent and aqueous phases by centrifugation. The aqueous phase was retained and filtered. Proteins were denatured by addition of urea and diluted to a concentration of 2 grams per liter protein in a 6 molar urea solution at pH 8. Target protein was refolded in batch mode by a ten-fold dilution into a refolding buffer. Initial purification of active, monomeric vaccine was performed by bind and elute anion exchange chromatography, with target protein and impurity elution effected by increasing salt concentration in a step-wise fashion. After filtration and pH adjustment of the AEX eluate to 6.8, active, monomeric vaccine was further purified by utilizing a hydroxyapatite (CHT) chromatography media in binding mode. Elution of impurities and target protein was effected by increasing phosphate concentration in steps. After filtration, the material was buffer-exchanged into a formulation buffer by TFF and dilution, filtered, aliquoted, and stored at - 70C. The resulting bulk drug substance was diluted to the target drug product concentration in the same formulation buffer and stored at -70C prior to administration.

### Manufacture of VAX128B

A pET24a plasmid encoding production of STF2R3.HA1-2 CA07 was used to transform commercially available *E. coli* BLR(DE3) cells. These cells were expanded to manufacture both research and cGMP master cell banks. To produce the target protein, a vial of banked cells was expanded in shaker flasks using synthetic media and grown to a specific optical density. A specific amount of cells were then transferred to a bioreactor containing a different synthetic media. The bioreactor was run under automatic control (T=30C, pH = 7, DO >= 30%) in batch mode until the glucose was exhausted. At that point a synthetic feed media was added to the bioreactor at a specific flow rate. After three hours, target protein production was initiated in the culture by addition of IPTG to a concentration of 2 mM. The cells were harvested four hours after induction and the cell paste separated from the conditioned media by centrifugation. After freezing and thawing, the cells were re-suspended in a Tris acetate buffer and lysed by high pressure homogenization. The lysate pellet was collected after centrifugation, re-suspended in a phosphate buffer, and collected again by subsequent centrifugation. The pellet resuspension and collection process was repeated and the washed pellet was solubilized in a 6M urea buffer at pH 8. After mixing, solubilized proteins were recovered by centrifugation and diluted to 2 grams protein per liter of solution. The target protein was refolded into its proper conformation by rapidly diluting the denatured proteins into a nine fold excess of refolding buffer under flow. The denatured protein solution and refolding buffer were mixed in line at a 1 to 9 flow rate ratio, held for an average of 30 minutes before loading on an anion exchange chromatography column. Active, monomeric vaccine was bound to the AEX media with target protein and impurity elution effected by increasing salt concentration in steps. After filtration, the anion exchange elution was diluted 10% with water and loaded onto a different anion exchange chromatography column for further purification. Elution of impurities and target protein was effected by increasing Tris/acetate concentration in steps. After filtration, the material was buffer-exchanged into a formulation buffer by TFF and dilution, filtered, aliquoted, and stored at -70C. The resulting bulk drug substance was diluted to the target drug product concentration in the same formulation buffer and stored at -70C prior to administration.

### Manufacture of VAX128C

A pET24a plasmid encoding production of STF2R3.2xHA1-2 CA07 was used to transform commercially available *E. coli* BLR(DE3) cells. These cells were expanded to manufacture both research and cGMP master cell banks. To produce the target protein, a vial of banked cells was expanded in shaker flasks using synthetic media and grown to a specific optical density. A specific amount of cells were then transferred to a bioreactor containing a synthetic media. The bioreactor was run under automatic control (T=30C, pH = 7, DO >= 30%) in batch mode until the glucose was exhausted. At that point a synthetic feed media was added to the bioreactor at a specific flow rate. After three hours, target protein production was initiated in the culture by addition of IPTG to a concentration of 2 mM. The cells were harvested four hours after induction and the cell paste separated from the conditioned media by centrifugation. After freezing and thawing, the cells were re-suspended in a Tris/acetate buffer and lysed by high pressure homogenization. The lysate pellet was collected after centrifugation, re-suspended in a phosphate buffer, and collected again by subsequent centrifugation. The pellet resuspension and collection process was repeated and the washed pellet was solubilized in a 6M urea buffer at pH 8. After mixing, solubilized proteins were recovered by centrifugation and diluted to 2 grams protein per liter of solution. Target protein was refolded in batch mode by a ten-fold dilution into a refolding buffer. Initial purification of active, monomeric vaccine was performed by bind and elute anion exchange chromatography, with impurity and target protein elution effected by increasing salt concentration in steps. After filtration and pH adjustment of the AEX eluate to 6.8, active, monomeric vaccine was further purified by utilizing a hydroxyapatite (CHT) chromatography media in binding mode. Elution of impurities and target protein was effected by increasing phosphate concentration in a step-wise fashion. After filtration, the material was buffer exchanged into a formulation buffer by TFF and dilution, filtered, aliquoted, and stored at -70C. The resulting bulk drug substance was diluted to the target drug product concentration in the same formulation buffer and stored at -70C prior to administration.

### Synthetic shake flask media

| Component | Amount |
|---|---|
| Water | 940 mL |
| KH₂PO₄ | 7.8 g/L |
| Citric Acid | 1.0 g/L |
| (NH₄)₂SO₄ | 2.33 g/L |
| Trace Metals Solution | 1.0 mL/L |
| Thiamine HCl | 0.01 g/L |
| Glucose | 10 g/L |
| MgSO₄ | 1 g/L |
| CaCl₂ | 0.04 g/L |
| Kanamycin | 0.0075 g/L |
| 10N NaOH | As needed to adjust final pH to 7.0-7.2 |

### Synthetic bioreactor media

| Component | Amount |
|---|---|
| Water | As needed to obtained calculated final volume |
| KH₂PO₄ | 2.2 g/L |
| (NH₄)₂SO₄ | 4.5 g/L |
| Citric Acid | 1.0 g/L |
| Trace Metals Solution | 1.0 mL/L |
| Thiamine HCl | 0.01 g/L |
| Antifoam | 100 µL per L |
| 10N NaOH | As needed to adjust base MRBR to pH to 6.0-6.5 |
| Glucose | 20 g/L |
| MgSO₄ | 1 g/L |
| CaCl₂ | 0.04 g/L |
| Kanamycin | 0.0075 g/L |
| 10N NaOH Solution | As needed to adjust final MRBR to pH to 6.0-6.5 |

### Synthetic feed media

| Component | Amount |
|---|---|
| Water | As needed to obtained calculated final volume |
| Dextrose | 180.0 g/L |
| L-Alanine | 40.0 g/L |
| KH₂PO₄ | 1.5 g/L |
| (NH₄)₂HPO₄ | 10.0 g/L |
| NaH₂PO₄ | 4.0 g/L |
| (NH₄)₂SO₄ | 5.0 g/L |
| Citric Acid | 4.0 g/L |
| Sulfuric Acid | As needed to adjust pH to 5.0-5.4 |
| MgSO₄ | 2.3 g/L |
| Trace Metals Solution | 6 mL/L |
| CaCl₂ | 2 g/L |
| Thiamine HCl | 0.01 g/L |
| FeSO₄ | 0.25 g/L |
| 10N NaOH Solution | As needed to adjust pH to 5.95-6.05 |

### Trace Metals Solution

| Component | Amount |
|---|---|
| Water | As needed to obtained calculated final volume |
| EDTA | 5.0 g/L |
| FeSO₄· 7H₂O | 10.0 g/L |
| ZnSO₄· 7H₂O | 2.0 g/L |
| MnSO₄· H₂O | 2.0 g/L |
| CoCl₂·6H₂O | 0.2 g/L |
| CuSO₄·SH₂O | 0.1 g/L |
| Na₂MoO₄· 2H₂O | 0.2 g/L |
| H₃BO₃ | 0.1 g/L |
| Sulfuric Acid | As needed to remove precipitate |

### Formulation buffer (pH7)

| Component | Amount |
|---|---|
| Tris | 10 mM |
| Histidine | 10 mM |
| Trehalose | 5% (w/v) |
| NaCl | 150 mM |
| Polysorbate-80 | 0.02% (w/v) |
| EDTA | 0.1 mM |
| Ethanol | 0.5% (v/v) |
| WFI | q.s. |

### Example 3

### Phase I Escalating Dose Ranging Study of Vax 128 A, B, C

A Phase I escalating dose ranging study to evaluate the safety and immunogenicity of VAX128 A, B, and C H1N1 influenza vaccine constructs in healthy adults 18-49 years of age and in adults ≥65 years of age was conducted. These 3 novel influenza vaccine constructs are comprised of the globular head of the HA1 domain of the A/California/07, Novel H1N1 (VAX128) genetically fused to the TLR5 ligand, flagellin, and produced in *E. coli.* In Vax128A the HA1 was fused to the C-terminus of flagellin, while in VAX128B HA1 replaced the D3 domain of flagellin and in VAX128C HA1 was replaced the D3 domain and was fused to the C-terminus. Vaccine was supplied in glass vials at either 20 µg/mL or 2 µg/mL, and diluted in dilution buffer (150mM NaCl with histidine and trehalose) to the final concentration on the day of administration. Study materials were prepared by non-blinded pharmacists and provided to blinded clinical staff. Vaccine was administered at a final volume of 0.5 ml by deep intramuscular injection in the deltoid muscle.

116 healthy adult subjects 18-49 and 100 healthy adults ≥ 65 years old were enrolled in a double blind, placebo controlled clinical trial conducted at two centers. Subjects received one of the 3 VAX128 vaccine constructs or placebo in a dose escalation study in a ratio of 3:3:3:1. Vaccines were administered IM at doses ranging from 0.5-20 µg. Subjects were followed for safety and sera were tested by hemagglutination-inhibition (HAI) against egg-grown virus on days 0, 7, 14, and 28. Serum C-reactive protein (CRP), cytokine levels, and anti-flagellin antibody were also assessed.

**Table 13. Number (%) of young adults subjects with local and systemic symptoms in VAX128-01, n=112**

| | VAX128A | | | | VAX128B | | VAX128C | | | Placebo |
|---|---|---|---|---|---|---|---|---|---|---|
| Dose ranges | <2.5 N=6 | 2.5 n=12 | 4 n=12 | 8 n=6 | < 16 n=18 | 16 n=12 | < 16 n=18 | 16 n=12 | 20 N=6 | 0 n=10 |
| Temp. > 99* F | 0 | 0 | 1(8) | 1(17) | 1(6) | 0 | 4(22) | 0 | 2 (33) | 0 |
| Local | | | | | | | | | | |
| None | 0 | 3(25) | 3(25) | 0 | 3(17) | 2(17) | 4(22) | 1(8) | 1(17) | 8(80) |
| Mild | 3(50) | 2 (17) | 4 (33) | 3(50) | 7(39) | 7(58) | 9 | 3(25) | 1 (17) | 2(20) |
| Moderate | 2(33) | 7(58) | 1(8) | 2(33) | 8(44) | 3(25) | 4(22) | 6(50) | 4(67) | 0 |
| Severe | 1(17) | 0 | 1(8) | 1(17) | 0 | 0 | 1(6) | 2(17) | 0 | 0 |
| Systemic | | | | | | | | | | |
| None | 3(50) | 9(75) | 11(92) | 5(83) | 12(67) | 9(75) | 12 (67) | 7 (58) | 5 (83) | 6(60) |
| Mild | 3(50) | 1(8) | 0 | 1(17) | 5(28) | 1(8) | 3 (17) | 2 (17) | 1 (17) | 3(30) |
| Moderate | 0 | 2 (17) | 0 | 0 | 1(6) | 1 (8) | 3 (17) | 1 (8) | 0 | 1(10) |
| Severe | 0 | 0 | 1(8) | 0 | 0 | 1(8) | 0 | 2(17) | 0 | 0 |
| CRP day 1 > 40 | 0 | 0 | 0 | 1(17) | 1(6) | 4(33) | 0 | 0 | 0 | 0 |
| CRP fold > 40 | 0 | 0 | 0 | 0 | 3 (17) | 1(8) | 1(6) | 0 | 0 | 0 |
| WBC increase | 0 | 1(8) | 0 | 0 | 0 | 2 (17) | 0 | 2(17) | 0 | 0 |
| LFT increase | 0 | 2 (17) | 0 | 0 | 1(6) | 2 (17) | 0 | 0 | 0 | 0 |
| IL-6 increase | 0 | 0 | 0 | 0 | 3(17) | 3(25) | 1(6) | 0 | 0 | 0 |

**Table14. Number (%) of subjects ≥ 65 with local and systemic symptoms or lab abnormities in VAX128-01, n=100**

| Dose ranges (µg) | VAX128A | | VAX1288 | | VAX128C | | Placebo |
|---|---|---|---|---|---|---|---|
| | 12.5-4 n=9 | 8 n=21 | 1.25-12 n=18 | 16 n=12 | 1.25-12 n=15 | 16-20 n=15 | 0 n=10 |
| Temp. > 99* F | 0 | 3(14) | 0 | 0 | 0 | 1(7) | 0 |
| Local | | | | | | | |
| None | 4 (44) | 10 (48) | 8 (44) | 2 (17) | 3(20) | 5 (33) | 10(100) |
| Mild | 2 (22) | 9 (43) | 8 (44) | 4 (33) | 10 (67) | 7 (47) | 0 |
| Moderate | 3 (33) | 2(10) | 2(11) | 6(50) | 2(13) | 3(20) | 0 |
| Severe | 0 | 0 | 0 | 0 | 0 | 0 | 0 |
| **Systemic** | | | | | | | |
| None | 9 (100) | 17 (81) | 13 (72) | 7 (58) | 13 (87) | 13 (87) | 10 (100) |
| Mild | 0 | 4(19) | 4(22) | 4(33) | 2(13) | 1(7) | 0 |
| Moderate | 0 | 0 | 0 | 1 (8) | 0 | 1 (7) | 0 |
| Severe | 0 | 0 | 1(6) | 0 | 0 | 0 | 0 |
| CRP day 1 > 40 | 0 | 1(5) | 1(6) | 1(8) | 1 (7) | 1 (7) | 0 |
| CRP fold > 40 | 0 | 0 | 0 | 2(17) | 1(7) | 1(7) | 0 |
| WBC increase | 0 | 3 (14) | 1(6) | 1 (8) | 1(7) | 2(13) | 0 |
| LFT increase | 1 (11) | 0 | 1(6) | 1 (8) | 0 | 0 | 0 |
| IL-6 increase | 0 | 0 | 1(6) | 1(8) | 0 | 0 | 0 |

**Table 15a GMT HAI titers, seroconversion (SC) and seroprotection (SP) rates among 112 subjects 18-49 years old who received a single IM dose of VAX128 (CA07) construct A, B or C (construct groups combined).**

| | | GMT HAI on day | | | | MFR on day | | | | | |
|---|---|---|---|---|---|---|---|---|---|---|---|
| Dose (µg) | No. of Subjects | 0 | 7 | 14 | 28 | 14 | 28 | SC | SP | SC% | SP% |
| placebo | 10 | 9 | 10 | 10 | 10 | 1 | 1 | 0 | 2 | 0 | 20 |
| 0.5 | 9 | 14 | 29 | 86 | 63 | 6 | 5 | 7 | 7 | 78 | 78 |
| 1.25 | 9 | 7 | 25 | 218 | 148 | 32 | 22 | 7 | 7 | 78 | 78 |
| 2.5 | 18 | 12 | 83 | 231 | 196 | 20 | 20 | 16 | 17 | 89 | 94 |
| 4 | 18 | 16 | 148 | 453 | 359 | 29 | 23 | 17 | 17 | 94 | 94 |
| 8 | 12 | 13 | 150 | 320 | 190 | 25 | 15 | 9 | 10 | 75 | 83 |
| 12 | 6 | 16 | 1280 | 1810 | 905 | 114 | 57 | 6 | 6 | 100 | 100 |
| 16 | 24 | 10 | 301 | 567 | 433 | 53 | 40 | 19 | 19 | 79 | 79 |
| 20 | 6 | 20 | 359 | 640 | 453 | 32 | 23 | 5 | 6 | 83 | 100 |

**Table 15b. GMT HAI titers, seroconversion (SC) and seroprotection (SP) rates among 100 subjects ≥ 65 years old who received a single IM dose of VAX128 (CA07) construct A, B or C (construct groups combined).**

| | | GMT HAI on day | | | | MFR on day | | | | | |
|---|---|---|---|---|---|---|---|---|---|---|---|
| Dose (µg) | No. of Subjects | 0 | 7 | 14 | 28 | D14 fold | D28 fold | SC | SP | SC% | SP% |
| placebo | 10 | 20 | 20 | 21 | 21 | 1 | 1 | 0 | 3 | 0 | 30 |
| 1.25 | 9 | 10 | 13 | 13 | 16 | 2 | 2 | 2 | 3 | 22 | 33 |
| 2.5 | 9 | 22 | 50 | 93 | 69 | 4 | 3 | 4 | 8 | 44 | 89 |
| 4 | 9 | 27 | 74 | 109 | 93 | 4 | 3 | 5 | 8 | 56 | 89 |
| 8 | 30 | 12 | 36 | 88 | 84 | 7 | 7 | 19 | 23 | 63 | 77 |
| 12 | 6 | 16 | 80 | 226 | 202 | 14 | 13 | 3 | 5 | 50 | 83 |
| 16 | 15 | 14 | 101 | 221 | 175 | 16 | 13 | 9 | 10 | 60 | 67 |
| 20 | 12 | 7 | 34 | 127 | 113 | 18 | 16 | 9 | 9 | 75 | 75 |

**Table 16 GMT HAI titers, seroconversion (SC) and seroprotection (SP) rates among 100 subjects ≥ 65 years old who received a single IM dose of VAX128 (CA07) construct A, B or C.**

| | | | GMT HAI on day | | | | MFR on day | | | | | |
|---|---|---|---|---|---|---|---|---|---|---|---|---|
| Vaccine | dose | Subj. | 0 | 7 | 14 | 28 | 14 | 28 | SC | SP | SC% | SP% |
| VAX128A | 1.25 | 3 | 16 | 25 | 40 | 40 | 2.5 | 2.5 | 1 | 2 | 33 | 67 |
| | 2.5-4 | 6 | 11 | 40 | 71 | 63 | 6.3 | 5.7 | 4 | 6 | 67 | 100 |
| | 8 | 21 | 13 | 26 | 56 | 56 | 4.4 | 4.4 | 11 | 15 | 52 | 71 |
| | | | | | | | | | | | | |
| VAX128B | 1.25 | 3 | 5 | 5 | 5 | 5 | 1.0 | 1.0 | 0 | 0 | 0 | 0 |
| | 2.5-4 | 6 | 22 | 45 | 80 | 80 | 3.6 | 3.6 | 3 | 5 | 50 | 83 |
| | 8-12 | 9 | 17 | 93 | 373 | 296 | 21.8 | 17.3 | 8 | 9 | 89 | 100 |
| | 16 | 12 | 8 | 53 | 143 | 107 | 17.0 | 12.7 | 7 | 7 | 58 | 58 |
| | | | | | | | | | | | | |
| VAX128C | 1.25 | 3 | 13 | 16 | 20 | 20 | 1.6 | 1.6 | 1 | 1 | 33 | 33 |
| | 2.5-4 | 6 | 57 | 127 | 180 | 101 | 3.2 | 1.8 | 2 | 5 | 33 | 83 |
| | 8-12 | 6 | 7 | 57 | 127 | 127 | 18.0 | 18.0 | 3 | 4 | 50 | 67 |
| | 16-20 | 15 | 12 | 70 | 202 | 184 | 16.8 | 15.3 | 11 | 12 | 73 | 80 |

**Table 17. Immune response by age cohort**

| | | GMT HAI on day | | | | MFR on day | | | | | |
|---|---|---|---|---|---|---|---|---|---|---|---|
| Age | Group | 0 | 7 | 14 | 28 | 14 | 28 | SC | SC% | SP | SP% |
| 65-69 | N=30 | 12.9 | 56.6 | 113.1 | 94.0 | 8.8 | 7.3 | 18 | 60 | 24 | 80 |
| 70-79 | N=46 | 10.0 | 26.6 | 66.8 | 61.0 | 6.7 | 6.1 | 24 | 52 | 30 | 65 |
| 80-90 | N=14 | 32.8 | 144.9 | 275.8 | 249.8 | 8.4 | 7.6 | 9 | 64 | 12 | 86 |

**Table 18. HAI Titers for elderly adults 65 and over by VAX128 construct and dose**

| | | | | HAI on day | | | | Fold on day | | | |
|---|---|---|---|---|---|---|---|---|---|---|---|
| Subject | Age | Vaccine | Dose | 0 | 7 | 14 | 28 | 14 | 28 | SC | SP |
| 1 | 69 | B | 1.25 | 5 | 5 | 5 | 5 | 1 | 1 | N | N |
| 2 | 76 | B | 1.25 | 5 | 5 | 5 | 5 | 1 | 1 | N | N |
| 3 | 78 | P | 0 | 10 | 10 | 10 | 10 | 1 | 1 | N | N |
| 4 | 71 | C | 2.5 | 10 | 40 | 80 | 20 | 8 | 2. | Y | Y |
| 5 | 79 | C | 1.25 | 10 | 10 | 10 | 10 | 1 | 1 | N | N |
| 6 | 65 | C | 1.25 | 20 | 40 | 80 | 80 | 4 | 4 | Y | Y |
| 7 | 66 | C | 2.5 | 40 | 80 | 40 | 40 | 1 | 1 | N | Y |
| 8 | 70 | A | 1.25 | 80 | 160 | 160 | 160 | 2 | 2 | N | Y |
| 9 | 72 | C | 1.25 | 10 | 10 | 10 | 10 | 1 | 1 | N | N |
| 10 | 79 | B | 2.5 | 5 | 5 | 10 | 5 | 2 | 1 | N | N |
| 11 | 74 | A | 2.5 | 10 | 20 | 160 | 160 | 16 | 16 | Y | Y |
| 12 | 67 | A | 2.5 | 40 | 80 | 80 | 20 | 2 | 0.5 | N | Y |
| 13 | 86 | A | 4 | 20 | 80 | 80 | 80 | 4 | 4 | Y | Y |
| 14 | 65 | A | 2.5 | 10 | 20 | 20 | 40 | 2 | 4 | N | Y |
| 15 | 68 | A | 4 | 5 | 40 | 160 | 160 | 32 | 32 | Y | Y |
| 16 | 74 | B | 4 | 5 | 40 | 80 | 40 | 16 | 8 | Y | Y |
| 17 | 84 | C | 2.5 | 20 | 640 | 2560 | 640 | 128 | 32 | Y | Y |
| 18 | 69 | A | 1.25 | 10 | 20 | 80 | 80 | 8 | 8 | Y | Y |
| 19 | 90 | B | 2.5 | 160 | 160 | 640 | 2560 | 4 | 16 | Y | Y |
| 20 | 68 | P | 0 | 20 | 10 | 10 | 10 | 0.5 | 0.5 | N | N |
| 21 | 72 | P | 0 | 40 | 40 | 40 | 40 | 1 | 1 | N | Y |
| 22 | 71 | B | 1.25 | 5 | 5 | 5 | 5 | 1 | 1 | N | N |
| 23 | 68 | B | 2.5 | 40 | 40 | 40 | 40 | 1 | 1 | N | Y |
| 24 | 77 | A | 1.25 | 5 | 5 | 5 | 5 | 1 | 1 | N | N |
| 25 | 65 | B | 8 | 80 | 2560 | 2560 | 2560 | 32 | 32 | Y | Y |
| 26 | 74 | C | 4 | 2560 | 2560 | 2560 | 2560 | 1 | 1 | N | Y |
| 27 | 65 | C | 4 | 5 | 5 | 5 | 5 | 1 | 1 | N | N |
| 28 | 83 | C | 12 | 5 | 640 | 2560 | 2560 | 512 | 512 | Y | Y |
| 29 | 85 | C | 4 | 320 | 160 | 320 | 160 | 1 | 0.5 | N | Y |
| 30 | 89 | B | 4 | 160 | 320 | 160 | 160 | 1 | 1 | N | Y |
| 31 | 67 | B | 8 | 5 | 80 | 320 | 160 | 64 | 32 | Y | Y |
| 32 | 84 | P | 0 | 40 | 80 | 80 | 80 | 2 | 2 | N | Y |
| 33 | 66 | B | 4 | 5 | 20 | 80 | 80 | 16 | 16 | Y | Y |
| 34 | 69 | A | 8 | 10 | 5 | 5 | 5 | 0.5 | 0.5 | N | N |
| 35 | 70 | B | 8 | 5 | 5 | 20 | 40 | 4 | 8 | Y | Y |
| 36 | 68 | A | 4 | 5 | 40 | 40 | 40 | 8 | 8 | Y | Y |
| 37 | 71 | C | 8 | 5 | 5 | 20 | 20 | 4 | 4 | N | N |
| 38 | 71 | B | 12 | 20 | 80 | 640 | 320 | 32 | 16 | Y | Y |
| 39 | 69 | A | 8 | 20 | 160 | 320 | 320 | 16 | 16 | Y | Y |
| 40 | 65 | B | 8 | 5 | 80 | 80 | 40 | 16 | 8 | Y | Y |
| 41 | 66 | A | 8 | 40 | 80 | 80 | 80 | 2 | 2 | N | Y |
| 42 | 70 | A | 8 | 10 | 40 | 160 | 160 | 16 | 16 | Y | Y |
| 43 | 67 | C | 16 | 40 | 2560 | 2560 | 2560 | 64 | 64 | Y | Y |
| 44 | 73 | A | 8 | 20 | 20 | 20 | 40 | 1 | 2 | N | Y |
| 45 | 69 | C | 8 | 5 | 80 | 160 | 320 | 32 | 64 | Y | Y |
| 46 | 80 | C | 12 | 40 | 40 | 40 | 40 | 1 | 1 | N | Y |
| 47 | 77 | C | 8 | 5 | 160 | 640 | 320 | 128 | 64 | Y | Y |
| 48 | 84 | A | 8 | 80 | 80 | 320 | 160 | 4 | 2 | Y | Y |
| 49 | 74 | B | 8 | 20 | 160 | 640 | 320 | 32 | 16 | Y | Y |
| 50 | 66 | A | 8 | 5 | 40 | 40 | 20 | 8 | 4 | Y | Y |
| 51 | 69 | B | 8 | 40 | 80 | 2560 | 2560 | 64 | 64 | Y | Y |
| 52 | 77 | A | 8 | 20 | 40 | 40 | 40 | 2 | 2 | N | Y |
| 53 | 75 | C | 16 | 160 | 320 | 320 | 320 | 2 | 2 | N | Y |
| 54 | 75 | P | 0 | 80 | 80 | 80 | 80 | 1 | 1 | N | Y |
| 55 | 74 | B | 12 | 40 | 40 | 40 | 40 | 1 | 1 | N | Y |
| 56 | 76 | B | 12 | 20 | 160 | 2560 | 2560 | 128 | 128 | Y | Y |
| 57 | 77 | C | 12 | 5 | 20 | 20 | 20 | 4 | 4 | N | N |
| 58 | 72 | P | 0 | 20 | 20 | 20 | 20 | 1 | 1 | N | N |
| 59 | 81 | 8 | 16 | 5 | 5 | 5 | 5 | 1 | 1 | N | N |
| 60 | 83 | C | 16 | 160 | 2560 | 2560 | 2560 | 16 | 16 | Y | Y |
| 61 | 77 | C | 20 | 5 | 5 | 10 | 10 | 2 | 2 | N | N |
| 62 | 84 | B | 16 | 40 | 640 | 2560 | 2560 | 64 | 64 | Y | Y |
| 63 | 66 | A | 8 | 160 | 320 | 320 | 160 | 2 | 1 | N | Y |
| 64 | 72 | B | 16 | 5 | 5 | 20 | 10 | 4 | 2 | N | N |
| 65 | 77 | C | 20 | 5 | 20 | 160 | 160 | 32 | 32 | Y | Y |
| 66 | 72 | C | 20 | 5 | 80 | 160 | 80 | 32 | 16 | Y | Y |
| 67 | 65 | A | 8 | 5 | 40 | 160 | 80 | 32 | 16 | Y | Y |
| 68 | 70 | A | 8 | 5 | 40 | 640 | 320 | 128 | 64 | Y | Y |
| 69 | 73 | A | 8 | 5 | 5 | 40 | 40 | 8 | 8 | Y | Y |
| 70 | 79 | C | 20 | 10 | 80 | 320 | 160 | 32 | 16 | Y | Y |
| 71 | 77 | B | 16 | 5 | 10 | 20 | 10 | 4 | 2 | N | N |
| 72 | 71 | A | 8 | 5 | 5 | 20 | 10 | 4 | 2 | N | N |
| 73 | 70 | C | 20 | 5 | 40 | 160 | 80 | 32 | 16 | Y | Y |
| 74 | 72 | A | 8 | 40 | 640 | 640 | 640 | 16 | 16 | Y | Y |
| 75 | 65 | B | 16 | 5 | 2560 | 2560 | 640 | 512 | 128 | Y | Y |
| 76 | 65 | P | 0 | 20 | 20 | 20 | 20 | 1 | 1 | N | N |
| 77 | 69 | B | 16 | 5 | 20 | 20 | 20 | 4 | 4 | N | N |
| 78 | 81 | A | 8 | 10 | 10 | 20 | 20 | 2 | 2 | N | N |
| 79 | 69 | P | 0 | 20 | 20 | 20 | 20 | 1 | 1 | N | N |
| 80 | 74 | C | 20 | 5 | 5 | 5 | 5 | 1 | 1 | N | N |
| 81 | 74 | C | 20 | 5 | 40 | 320 | 640 | 64 | 128 | Y | Y |
| 82 | 69 | C | 20 | 5 | 5 | 10 | 5 | 2 | 1 | N | N |
| 83 | 68 | B | 16 | 20 | 80 | 2560 | 2560 | 128 | 128 | Y | Y |
| 84 | 68 | B | 16 | 10 | 160 | 2560 | 2560 | 256 | 256 | Y | Y |
| 85 | 85 | B | 16 | 20 | 640 | 2560 | 2560 | 128 | 128 | Y | Y |
| 86 | 73 | A | 8 | 5 | 20 | 40 | 80 | 8 | 16 | Y | Y |
| 87 | 71 | P | 0 | 5 | 5 | 5 | 5 | 1 | 1 | N | N |
| 88 | 72 | C | 20 | 20 | 20 | 80 | 80 | 4 | 4 | Y | Y |
| 89 | 68 | A | 8 | 80 | 80 | 320 | 320 | 4 | 4 | Y | Y |
| 90 | 71 | C | 20 | 40 | 160 | 160 | 320 | 4 | 8 | Y | Y |
| 91 | 70 | C | 20 | 5 | 80 | 2560 | 2560 | 512 | 512 | Y | Y |
| 92 | 79 | B | 16 | 10 | 40 | 80 | 40 | 8 | 4 | Y | Y |
| 93 | 79 | B | 16 | 5 | 5 | 5 | 5 | 1 | 1 | N | N |
| 94 | 67 | A | 8 | 5 | 5 | 10 | 10 | 2 | 2 | N | N |
| 95 | 74 | C | 20 | 5 | 320 | 2560 | 2560 | 512 | 512 | Y | Y |
| 96 | 81 | B | 16 | 5 | 40 | 40 | 40 | 8 | 8 | Y | Y |
| 97 | 74 | A | 8 | 5 | 5 | 5 | 80 | 1 | 16 | Y | Y |
| 98 | 75 | A | 8 | 5 | 5 | 5 | 5 | 1 | 1 | N | N |
| 99 | 72 | P | 0 | 10 | 10 | 20 | 20 | 2 | 2 | N | N |
| 100 | 79 | A | 8 | 5 | 5 | 20 | 20 | 4 | 4 | N | N |

**Table 19. Antibody response after one or two doses of the egg-based novel H1N1 (CA09) as measured by the HAI assay**

| Product, Author | CSL vaccine, Greenberg | | | | Novartis vaccine, Clark | |
|---|---|---|---|---|---|---|
| Age group | 18-49 | 50-64 | 18-49 | 50-64 | 18-49 | 18-49 |
| Dose (ug) | 15 | 15 | 30 | 30 | 7.5 | 7.5 |
| Adjuvant | none | none | none | none | MF59 | M F59 |
| No. doses and Regimen | 1, Day 0 | 1, Day 0 | 1, Day 0 | 1, Day 0 | 1, Day 0 | 2, Day 0 & 7 |
| No. subjects | n=58 | n=62 | n=62 | n=58 | n=25 | n=26 |
| HAI Day 0 | 21 | 19 | 20 | 13 | 6 | 7 |
| HAI Day 21 | 307 | 157 | 514 | 174 | 172 | 282 |
| Mean fold resp. | 14 | 8 | 25 | 13 | 28 | 59 |
| Seroconversion | 76 | 66 | 82 | 74 | 76 | 92 |
| Seroprotection | 100 | 94 | 98 | 88 | 80 | 96 |

In young adults, the maximum tolerated dose (MTD) for VAX128A was 8 µg and VAX128B 16µg. VAX128C was safe at 20µg, the highest dose tested (Table 13). Serum antibody responses were seen by HAI after doses as low as 0.5 µg. Doses of 1.25 µg to 2.5 µg induced a GMT of 1:250 with over 90% seroconversion and seroprotection (Table 15a and 15b). In adults ≥ 65 years, all three vaccines were safe at the highest doses tested of 8 µg, 12 µg and 20 µg for VAX128A, B and C, respectively (Table 14). Doses of 2.5 to 4 µg were associated reciprocal HAI titers of 70-180 and doses of 8-16 µg were associated HAI titers of 60 to 370 (Table 16). Table 17 shows the immune response by age cohort and table 18 shows the response for each individual tested that was 65 year of age and older.

Altering the configuration of the HA1 and flagellin was used to produce influenza vaccines that are safe with a large therapeutic window. VAX128C with two copies of the HA1 globular head had the highest MTD and was the most immunogenic. The globular head of the influenza HA expressed in a prokaryotic system was able to induce a functional antibody response. Vigorous responses were seen at relatively low doses of HA antigen indicating that the addition of flagellin provided a substantial adjuvanting effect.

Within this disclosure, any indication that a feature is optional is intended provide adequate support (e.g., under 35 U.S.C. 112 or Art. 83 and 84 of EPC) for claims that include closed or exclusive or negative language with reference to the optional feature. Exclusive language specifically excludes the particular recited feature from including any additional subject matter. For example, if it is indicated that A can be drug X, such language is Intended to provide support for a claim that explicitly specifies that A consists of X alone, or that A does not include any other drugs besides X. "Negative" language explicitly excludes the optional feature itself from the scope of the claims. For example, if it is indicated that element A can include X, such language is intended to provide support for a claim that explicitly specifies that A does not include X. Non-limiting examples of exclusive or negative terms include "only," "solely," "consisting of," "consisting essentially of," "alone," "without", "in the absence of (e.g., other items of the same type, structure and/or function)" "excluding," "not including", "not", "cannot," or any combination and/or variation of such language.

Similarly, referents such as "a," "an," "said," or "the," are intended to support both single and/or plural occurrences unless the context indicates otherwise. For example "a dog" is intended to include support for one dog, no more than one dog, at least one dog, a plurality of dogs, etc. Non-limiting examples of qualifying terms that indicate singularity include "a single", "one," "alone", "only one," "not more than one", etc. Non-limiting examples of qualifying terms that indicate (potential or actual) plurality include "at least one," "one or more," "more than one," "two or more," "a multiplicity," "a plurality," "any combination of," "any permutation of," "any one or more of," etc. Claims or descriptions that include "or" between one or more members of a group are considered satisfied if one, more than one, or all of the group members are present in, employed in, or otherwise relevant to a given product or process unless indicated to the contrary or otherwise evident from the context.

Where ranges are given herein, the endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

## Claims

1. A fusion protein comprising at least a portion of at least one flagellin and at least one influenza antigen for use in stimulating protective immunity against infection consequent to exposure of a human who is ≥ 65 years old, to a source of influenza antigen, wherein fusion of the at least the portion of the flagellin and the at least one influenza antigen generates the fusion protein that is capable of achieving a seroresponse rate of at least 50% and the immunogenicity is greater than the at least one influenza antigen alone.

2. The fusion protein for use according to claim 1, wherein the dose is at least one dose selected from the group consisting of a 0.5 µg dose, 1 µg dose, 2 µg dose, 3 µg dose, 4 µg dose, 5 µg dose, 6 µg dose, 7 µg dose, 8µg dose, 10µg dose, 15µg dose, 20µg dose, 25µg dose and a 30µg dose.

3. The fusion protein for use according to claim 1, further including the step of administering at least one subsequent dose of the fusion protein to the human.

4. The fusion protein for use according to claim 1, wherein the fusion protein includes SEQ ID No. 1.

5. A fusion protein for use according to claim 1, wherein the influenza antigen is associated with a particle.

6. The fusion protein for use according to claim 5, wherein the particle is a virosome, liposome, biodegradable microsphere, self-assembling peptide nanoparticle, virus-like particle or combinations thereof.

7. The polypeptide sequence as set forth in Seq ID 3.

8. The polypeptide sequence as set forth in Seq ID 5.

9. The polypeptide sequence as set forth in Seq ID 7.

10. The fusion protein for use of any of claims 1, 2, 3, 5 or 6, wherein the influenza antigen is an influenza A antigen, an influenza B antigen or an influenza C antigen.

11. The fusion protein for use of any of claims 1, 2, 3, 5, 6 or 10, wherein the influenza antigen is a hemagglutinin influenza antigen.

12. The fusion protein for use of any of claims 1, 2, 3, 5, 6, 10 or 11, wherein the dose administered to the human who is ≥ 65 years old is about a 3 µg to about a 50 µg dose, about a 4 µg to about a 50 µg dose or about a 5 µg to about a 50 µg dose.

## Patentansprüche

1. Fusionsprotein, das mindestens einen Teil mindestens eines Flagellins und mindestens ein Influenza-Antigen umfasst, zur Verwendung beim Stimulieren einer Schutzimmunität gegen eine Infektion infolge einer Aussetzung eines Menschen, der ≥ 65 Jahre alt ist, gegenüber einer Influenza-Antigenquelle, wobei eine Fusion des mindestens einen Teils des Flagellins und des mindestens einen Influenza-Antigens das Fusionsprotein erzeugt, das eine Seroresponserate von mindestens 50 % erzielen kann, und die Immunogenität größer als die von dem mindestens einen Antigen allein ist.

2. Fusionsprotein zur Verwendung nach Anspruch 1, wobei die Dosis mindestens eine Dosis ist, die aus der Gruppe bestehend aus einer 0,5-µg-Dosis, 1-µg-Dosis, 2-µg-Dosis, 3-µg-Dosis, 4-µg-Dosis, 5-µg-Dosis, 6-µg-Dosis, 7-µg-Dosis, 8-µg-Dosis, 10-µg-Dosis, 15-µg-Dosis, 20-µg-Dosis, 25-µg-Dosis und einer 30-µg-Dosis ausgewählt ist.

3. Fusionsprotein zur Verwendung nach Anspruch 1, das weiterhin den Schritt des Verabreichens mindestens einer Folgedosis des Fusionsproteins an den Menschen beinhaltet.

4. Fusionsprotein zur Verwendung nach Anspruch 1, wobei das Fusionsprotein SEQ ID Nr. 1 beinhaltet.

5. Fusionsprotein zur Verwendung nach Anspruch 1, wobei das Influenza-Antigen mit einem Partikel assoziiert ist.

6. Fusionsprotein zur Verwendung nach Anspruch 5, wobei es sich bei dem Partikel um ein Virosom, ein Liposom, eine biologisch abbaubare Mikrosphäre, ein selbstassemblierendes Peptidnanopartikel, ein virusartiges Partikel oder Kombinationen davon handelt.

7. Wie in SEQ ID 3 dargelegte Polypeptidsequenz.

8. Wie in SEQ ID 5 dargelegte Polypeptidsequenz.

9. Wie in SEQ ID 7 dargelegte Polypeptidsequenz.

10. Fusionsprotein zur Verwendung nach einem der Ansprüche 1, 2, 3, 5 oder 6, wobei das Influenza-Antigen ein Influenza-A-Antigen, ein Influenza-B-Antigen oder ein Influenza-C-Antigen ist.

11. Fusionsprotein zur Verwendung nach einem der Ansprüche 1, 2, 3, 5, 6 oder 10, wobei das Influenza-Antigen ein Hämagglutin-Influenza-Antigen ist.

12. Fusionsprotein zur Verwendung nach einem der Ansprüche 1, 2, 3, 5, 6, 10 oder 11, wobei die an den Menschen, der ≥ 65 Jahre alt ist, verabreichte Dosis eine etwa 3-µg- bis eine etwa 50-µg-Dosis, eine etwa 4-µg- bis eine etwa 50-µg-Dosis oder eine etwa 5-µg- bis eine etwa 50-µg-Dosis ist.

## Revendications

1. Protéine de fusion comprenant au moins une partie d'au moins une flagelline et au moins un antigène de la grippe pour utilisation dans la stimulation d'immunité protectrice contre une infection résultant de l'exposition d'un être humain âgé de ≥ 65 ans à une source d'antigène de la grippe, dans laquelle la fusion de la au moins une partie de la flagelline et du au moins un antigène de la grippe génère la protéine de fusion qui est capable de réaliser un taux de réponse sérologique d'au moins 50 % et où l'immunogénicité est supérieure à l'au moins un antigène de la grippe seul.

2. Protéine de fusion pour utilisation selon la revendication 1, dans laquelle la dose est au moins une dose sélectionnée dans le groupe constitué par une dose de 0,5 µg, une dose de 1 µg, une dose de 2 µg, une dose de 3 µg, une dose de 4 µg, une dose de 5 µg, une dose de 6 µg, une dose de 7 µg, une dose de 8 µg, une dose de 10 µg, une dose de 15 µg, une dose de 20 µg, une dose de 25 µg et une dose de 30 µg.

3. Protéine de fusion pour utilisation selon la revendication 1, comprenant en outre l'étape d'administration d'au moins une dose ultérieure de la protéine de fusion à l'être humain.

4. Protéine de fusion pour utilisation selon la revendication 1, dans laquelle la protéine de fusion comprend la SEQ ID N° 1.

5. Protéine de fusion pour utilisation selon la revendication 1, dans laquelle l'antigène de la grippe est associée à une particule.

6. Protéine de fusion pour utilisation selon la revendication 5, dans laquelle la particule est un virosome, un liposome, une microsphère biodégradable, une nanoparticule de peptide capable d'auto-assemblage, une particule pseudovirale ou des combinaisons de ces derniers.

7. Séquence polypeptidique comme défini à Seq ID 3.

8. Séquence polypeptidique comme défini à Seq ID 5.

9. Séquence polypeptidique comme défini à Seq ID 7.

10. Protéine de fusion pour utilisation selon l'une quelconque des revendications 1, 2, 3, 5 ou 6, dans laquelle l'antigène de la grippe est un antigène de la grippe A, un antigène de la grippe B ou un antigène de la grippe C.

11. Protéine de fusion pour utilisation selon l'une quelconque des revendications 1, 2, 3, 5, 6 ou 10, dans laquelle l'antigène de la grippe est un antigène de la grippe hémagglutinine.

12. Protéine de fusion pour utilisation selon l'une quelconque des revendications 1, 2, 3, 5, 6, 10 ou 11, dans laquelle la dose administrée à l'être humain âgé de ≥ 65 ans est une dose d'environ 3 µg à environ 50 µg, une dose d'environ 4 µg à environ 50 µg ou une dose d'environ 5 µg à environ 50 µg.
